(19) Europäisches Patentamt
European Patent Office
Office européen des brevets

(11) EP 4 806 890 A1

(12) # EUROPEAN PATENT APPLICATION
published in accordance with Art. 153(4) EPC

(43) Date of publication:
**16.09.2026 Bulletin 2026/38**

(21) Application number: **24887950.4**

(22) Date of filing: **05.11.2024**

(51) International Patent Classification (IPC):
***C07D 401/14*** *(2006.01)* ***A61K 31/443*** *(2006.01)*
***A61K 31/444*** *(2006.01)* ***A61P 35/00*** *(2006.01)*

(52) Cooperative Patent Classification (CPC):
**A61K 31/443; A61K 31/444; A61P 35/00; C07D 401/14**

(86) International application number:
**PCT/CN2024/129978**

(87) International publication number:
**WO 2025/098338 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.11.2023 CN 202311475987**

(71) Applicant: **Abbisko Therapeutics Co., Ltd.**
**Shanghai 201203 (CN)**

(72) Inventors:
- **SONG, Jiaqi**
**Shanghai 201203 (CN)**
- **CHEN, Zhao**
**Shanghai 201203 (CN)**
- **ZHANG, Zhen**
**Shanghai 201203 (CN)**
- **YU, Hongping**
**Shanghai 201203 (CN)**

(74) Representative: **dompatent**
**Partnerschaft von**
**Patentanwälten und Rechtsanwälten mbB**
**Deichmannhaus am Dom**
**Bahnhofsvorplatz 1**
**50667 Köln (DE)**

(54) **CRYSTALLINE COMPOUND OR BASIC SALT THEREOF, PREPARATION METHOD THEREFOR, AND USE THEREOF**

(57) The present invention relates to a crystalline compound or a basic salt thereof, a preparation method therefor, and a use thereof. The compound is a PD-1/PD-L1 inhibitor having a compound structure as represented by the following formula (I). The crystalline compound has moderate hygroscopicity, improved solubility, good crystallinity, and stable physicochemical properties, and the basic salt of the crystalline compound has good crystallinity, good solubility, and stable physicochemical properties, meets industrial production requirements, and satisfies the need for the development of clinical pharmaceutical formulations. The crystalline compound or the basic salt thereof in the present invention can be widely applied to the preparation of drugs for treating PD-1/PD-L1 signaling pathway-mediated tumors, immune-related diseases and disorders, contagious diseases, infectious diseases, or metabolic diseases.

(I)



Processed by Luminess, 75001 PARIS (FR)

## Description

## TECHNICAL FIELD

The present invention belongs to the field of drug development and particularly relates to a crystalline PD-1/PD-L1 inhibitor compound or basic salt, preparation method therefor and use thereof.

## BACKGROUND

The immune system plays a very important role in controlling and eliminating diseases, such as cancer. However, tumor cells are often able to develop a strategy for escaping or suppressing the monitoring of the immune system to promote their malignant growth. One very important mechanism is to change the expression of co-stimulatory and co-inhibitory immune checkpoint molecules on immunocytes. Blocking the signal pathway of immune checkpoint molecules, such as PD-1, has been shown to be an extremely promising and effective therapeutic means.

Programmed cell death protein 1 (PD-1), also known as CD279, is a receptor expressed on the surfaces of activated T cells, natural killer T cells, B cells and macrophages. Its structure contains an extracellular domain similar to an immunoglobulin variable region, a transmembrane domain and an intracellular domain, where the intracellular domain contains two phosphorylation sites located in an immunoreceptor tyrosine kinase-based inhibitory domain and an immunoreceptor tyrosine kinase-based transduction domain, suggesting that PD-1 can down-regulate T cell receptor-mediated signal pathways.

PD-1 has two ligands: PD-L1 and PD-L2, and they are different in their expression profile. The expression of PD-L1 will be up-regulated in macrophages and dendritic cells after treatment with lipopolysaccharide (LPS) and granulocyte-macrophage colony-stimulating factor (GM-CSF), and will also be up-regulated in T cells and B cells after stimulation of T cell receptor and B cell receptor signal pathways. PD-L1 is also highly expressed in almost all tumor cells, and the expression will be up-regulated after stimulation of interferon (IFN) gamma. As a matter of fact, the expression of PD-L1 in a variety of tumors is considered to have prognostic relevance; in contrast, the expression of PD-L2 is relatively concentrated, mainly on dendritic cells.

When T cells expressing PD-1 come into contact with cells expressing the ligands of PD-1, those antigen-stimulated functional activities, such as cell proliferation, cytokine release and cell lysis activity, are all inhibited. Therefore, the interaction between PD-1 and ligands thereof can function as an intrinsic negative feedback regulation mechanism to prevent T cell hyperactivation during infection, immune tolerance or tumorigenesis, thus reducing the occurrence of autoimmune diseases and promoting self-tolerance. Long-term antigenic stimulation, e.g., in tumor or long-term infection, can cause T cells to express high levels of PD-1 and make them inactive and nonfunctional in these long-term reactions with antigens, which is also referred to as T cell exhaustion. For B cells, there are also inhibitory effects caused by PD-1 and its ligands and corresponding functional exhaustion.

Some evidence from preclinical animal studies indicates that PD-1 and ligands thereof can down-regulate the immunoreaction. PD-1-deficient mice will develop lupus erythematosus-like acute proliferative glomerulonephritis and dilated cardiomyopathy. Utilizing the antibody of PD-L1 to block PD-1/PD-L1 interaction has been shown to be able to restore and enhance T cell activation in many systems. The monoclonal antibody of PD-L1 can also benefit patients with advanced cancers. In some preclinical animal tumor models, it was also shown that blocking the signal pathway of PD-1/PD-L1 with a monoclonal antibody can enhance immunoreaction and lead to immunoreactions to a series of histologically significantly different tumors. With the long-term infection LCMV model, the interaction between PD-1 and PD-L1 has been found to be able to inhibit the activation and proliferation of virus-specific CD8 T cells and the acquisition of effector cell functions. Besides being capable of enhancing the immunoreaction to long-term antigens, blocking the pathway of PD-1/PD-L1 was also discovered to be able to enhance response to vaccines, including response to a therapeutic vaccine in long-term infection.

These results indicate that small-molecule inhibitors that target and block the PD-1/PD-L1 interaction can be an effective therapeutic means for blocking the PD-1/PD-L1-mediated inhibitory signal pathway to enhance or restore the function of T cells, and will have very good efficacy in immunotherapy for a variety of cancers and other immunity-related diseases.

During long-term research, Abbisko Therapeutics Co., Ltd. found a small-molecule compound having the effect of inhibiting PD-1/PD-L1 (WO2019149183A1, international publication date: August 8, 2019), a representative compound of which is shown below:

(I)

.

The name of the compound is (2*S*,2'*S*)-1,1'-(((((2,2'-dimethyl-[1,1'-biphenyl]-3,3'-diyl)bis(azanediyl))bis(carbonyl))bis(4-cyclopropylpyridine-6,3-diyl))bis(methylene))bis(piperidine-2-carboxylic acid) (the compound of formula (I)). The compound has a strong inhibitory effect on the protein interaction of PD-1/PD-L1, and the inhibitory effect can enhance or restore the activation of T cells at the cellular level. The compound can satisfy the requirements of target and immune therapy for tumors, immunity-related diseases and disorders, communicable diseases, infectious diseases or metabolic diseases, etc., at this stage in China and other countries.

During later pharmaceutical research, it was found that the compound of formula (I) reported in WO2019149183A1 is a trifluoroacetate freeze-dried amorphous compound that has poor solid-state properties, is very hygroscopic, has poor solubility and is not suitable for clinical formulation development. Therefore, in order to satisfy the needs of clinical research and the launch of drug formulations, there is an urgent need to develop an aggregate form suitable for drug development to overcome the defects in the prior art.

## SUMMARY

To solve the problems in the prior art, the inventors intensively studied different aggregate forms of the compound of formula (I) and developed several crystalline free forms of the compound or basic salts thereof through a large number of salt form and crystalline form screening experiments. The crystalline free forms of the compound greatly improve physicochemical properties, such as hygroscopicity, solubility and physicochemical stability, of the compound of formula (I), and the crystalline basic salts of the compound greatly improve physicochemical properties such as solubility and physicochemical stability. The crystalline free forms of the compound or the basic salts thereof have improved bioavailability, meet the requirements for industrial production and can satisfy the need to develop clinical pharmaceutical formulations. The crystalline forms of the compound or the basic salts thereof have very important clinical application value and are expected to be developed into new-generation PD-1/PD-L1 small-molecule inhibitors soon.

A first aspect of the present invention provides a crystalline basic salt of a compound of formula (I):

(I)

,

wherein the basic salt is selected from the group consisting of lithium salt, sodium salt, potassium salt, magnesium salt, calcium salt and ammonium salt.

As a preferred embodiment, the crystalline basic salt of the compound of formula (I) is a sodium salt.

As a further preferred embodiment, each molecule of the crystalline sodium salt of the compound of formula (I) comprises the compound of formula (I) in free form and the sodium atom in a molar ratio of 1:1 or 1:2, wherein the sodium salt is referred to as a monosodium salt when the molar ratio is 1:1, and the sodium salt is referred to as a disodium salt when the molar ratio is 1:2.

As a more further preferred embodiment, each molecule of the crystalline sodium salt of the compound of formula (I) comprises the compound of formula (I) in free form and the sodium atom in a molar ratio of 1:2.

As a preferred embodiment, the crystalline sodium salt of the compound of formula (I) is an anhydrate or a hydrate.

As a further preferred embodiment, each molecule of the crystalline sodium salt hydrate of the compound of formula (I) comprises the compound of formula (I) in free form and the water molecule in a molar ratio of 1.0:(0.1-10.0), wherein the hydrate is referred to as a hemihydrate when the molar ratio is 1.0:0.5, the hydrate is referred to as a monohydrate when the molar ratio is 1.0:1.0, and the hydrate is referred to as a dihydrate when the molar ratio is 1.0:2.0; and following this pattern, subsequent ratios are termed trihydrate, tetrahydrate, pentahydrate, and so on.

As a more further preferred embodiment, each molecule of the crystalline sodium salt hydrate of the compound of

formula (I) comprises the compound of formula (I) in free form and the water molecule in a molar ratio of 1.0:(1.0-5.0).

As a more further preferred embodiment, each molecule of the crystalline sodium salt hydrate of the compound of formula (I) comprises the compound of formula (I) in free form and the water molecule in a molar ratio of 1.0:4.0 or 1.0:2.0.

As a more further preferred embodiment, the crystalline basic salt of the compound of formula (I) is a crystalline form A of disodium salt hydrate having an X-ray powder diffraction (XRPD) pattern comprising five or more peaks at angles of diffraction (2θ) of 8.60±0.2°, 9.97±0.2°, 12.92±0.2°, 15.03±0.2°, 17.62±0.2°, 17.93±0.2°, 20.04±0.2°, 22.07±0.2°, 23.18±0.2°, 23.60±0.2° and 27.06±0.2°.

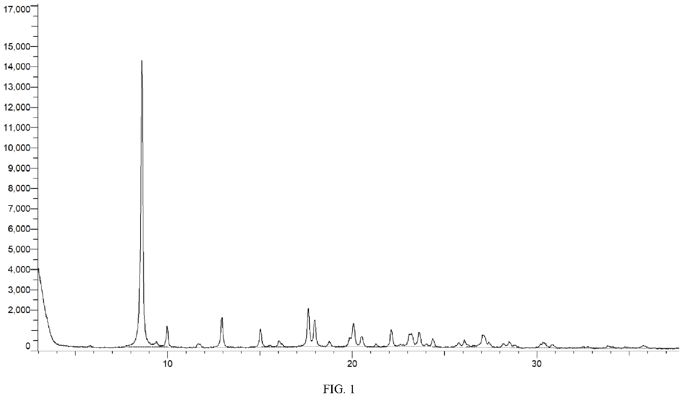

As the most preferred embodiment, the X-ray powder diffraction (XRPD) pattern of the crystalline form A of disodium salt hydrate comprises peaks substantially identical (±0.2°) to those at angles of diffraction (2θ) shown in FIG. 1, and X-ray powder diffraction data thereof are shown in Table 1:

Table 1

| 2θ (°) | Intensity% | 2θ (°) | Intensity% |
|---|---|---|---|
| 8.60 | 100.0% | 24.35 | 4.0% |
| 9.38 | 1.3% | 25.77 | 2.6% |
| 9.97 | 6.9% | 26.08 | 2.8% |
| 11.76 | 0.7% | 27.06 | 6.8% |
| 12.92 | 11.1% | 27.40 | 2.1% |
| 15.03 | 7.5% | 28.20 | 1.6% |
| 15.49 | 1.2% | 28.46 | 2.8% |
| 16.01 | 2.7% | 28.80 | 1.3% |
| 17.62 | 17.6% | 30.32 | 3.0% |
| 17.93 | 15.5% | 30.81 | 1.5% |
| 18.78 | 1.7% | 32.78 | 0.6% |
| 19.83 | 5.0% | 33.85 | 1.2% |
| 20.04 | 11.9% | 35.80 | 1.3% |
| 20.46 | 5.9% | 37.68 | 0.4% |
| 21.24 | 1.3% | 38.21 | 1.1% |
| 22.07 | 8.9% | 38.82 | 0.3% |
| 22.64 | 0.4% | 39.88 | 1.3% |
| 23.18 | 7.2% | 40.78 | 0.5% |
| 23.60 | 7.0% | 43.21 | 0.3% |
| 24.02 | 1.0% | 44.24 | 0.5% |

The crystalline form is designated crystalline form A of disodium salt hydrate.

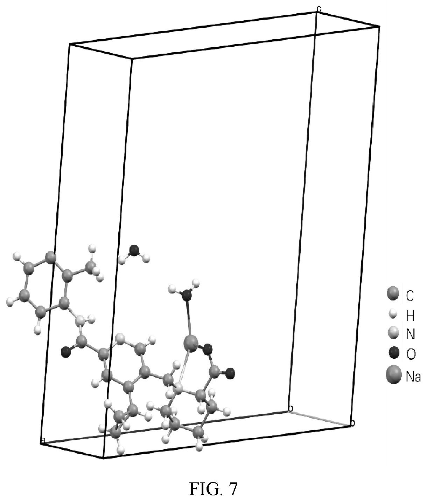

As the most preferred embodiment, the crystalline basic salt of the compound of formula (I) is a crystalline form A of disodium salt hydrate, wherein a unit cell of the crystalline form A of disodium salt hydrate is a monoclinic crystal system with the following unit cell parameters: a = 20.00(5) Å, b = 5.798(16) Å, c = 20.44(11) Å, α = 90°, β = 94.8(2)°, γ = 90° and a unit cell volume V of 2362(15) Å3. Z' is 0.5, and an asymmetric unit consists of 0.5 API anions, 1 sodium ion and 2 water molecules. The structure of a unit cell of a single crystal thereof is shown in FIG. 7.

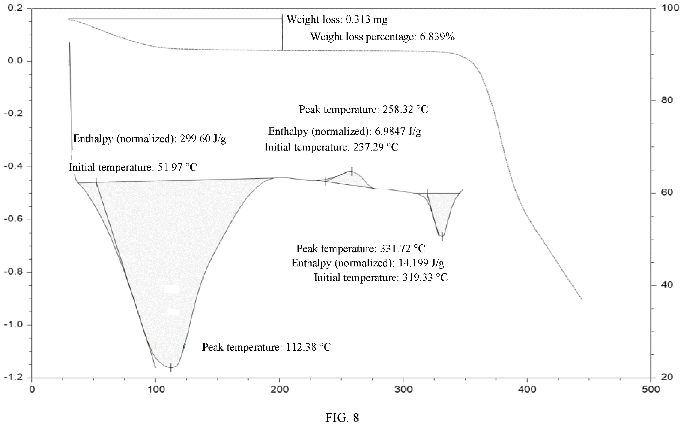

As the most preferred embodiment, the crystalline basic salt of the compound of formula (I) is a crystalline form A of disodium salt hydrate having DSC/TGA graphs that are substantially those shown in FIG. 8.

As a more further preferred embodiment, the crystalline basic salt of the compound of formula (I) is a crystalline form B of disodium salt anhydrate having an X-ray powder diffraction (XRPD) pattern comprising five or more peaks at angles of diffraction (2θ) of 6.60±0.2°, 7.46±0.2°, 12.43±0.2°, 13.32±0.2°, 13.63±0.2°, 15.54±0.2°, 17.32±0.2°, 18.68 ±0.2° and 21.73±0.2°.

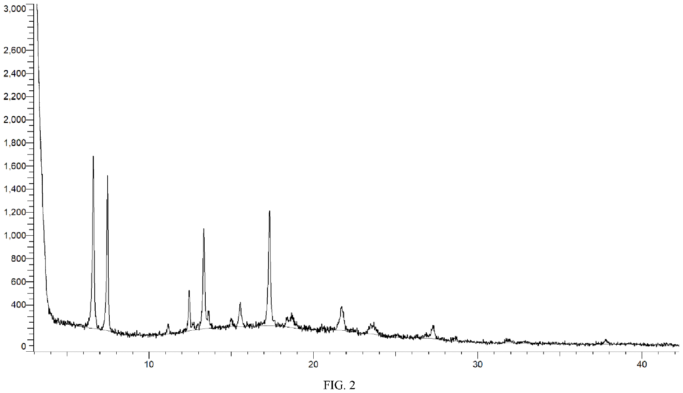

As the most preferred embodiment, the X-ray powder diffraction (XRPD) pattern of the crystalline form B of disodium salt anhydrate comprises peaks substantially identical (±0.2°) to those at angles of diffraction (2θ) shown in FIG. 2, and X-ray powder diffraction data thereof are shown in Table 2:

Table 2

| 2θ (°) | Intensity% | 2θ (°) | Intensity% |
|---|---|---|---|
| 6.60 | 100.0% | 18.41 | 6.8% |
| 7.46 | 89.9% | 18.68 | 8.4% |
| 11.16 | 4.1% | 20.51 | 3.1% |
| 12.43 | 23.3% | 21.73 | 13.7% |
| 13.32 | 58.0% | 23.57 | 3.5% |
| 13.63 | 10.3% | 27.26 | 5.6% |
| 14.99 | 4.8% | 28.64 | 2.1% |
| 15.54 | 13.3% | 31.83° | 2.6% |
| 17.32 | 66.6% | 37.82 | 1.9% |

The crystalline form is designated crystalline form B of disodium salt anhydrate and has a melting point of 316.4 °C.

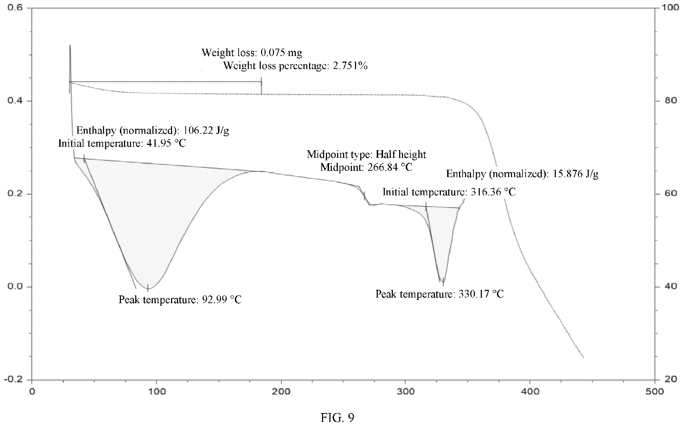

As the most preferred embodiment, the crystalline basic salt of the compound of formula (I) is a crystalline form B of disodium salt anhydrate having DSC/TGA graphs that are substantially those shown in FIG. 9.

A second aspect of the present invention provides a preparation method for the aforementioned crystalline basic salt of the compound of formula (I), comprising the following steps:

1) dissolving or dispersing the compound of formula (I) in free form in water or a suitable organic solvent, and adding a basic solution to the above system; or adding the compound of formula (I) in free form to a basic solution; and
2) collecting a solid product precipitated during the above salt-forming reaction, or creating a degree of supersaturation in the salt-forming system to obtain a crystalline product;

wherein the basic salt is selected from the group consisting of lithium salt, sodium salt, potassium salt, magnesium salt, calcium salt and ammonium salt.

As a preferred embodiment, the basic solution is selected from the group consisting of solutions of lithium hydroxide, sodium hydroxide, potassium hydroxide, magnesium hydroxide, calcium hydroxide and ammonia in water or in an organic solvent.

As a preferred embodiment, methods for creating the degree of supersaturation in the salt-forming system in step 2) of the preparation method include one or more of the following: adding a seed crystal, volatilizing the solvent, adding an anti-solvent and cooling.

As a preferred embodiment, the organic solvent during salt formation in step 1) of the preparation method is selected from the group consisting of alcohols, chloroalkanes, ketones, ethers, cyclic ethers, esters, alkanes, cycloalkanes, benzenes, amides and sulfoxides, and mixtures thereof and aqueous solutions thereof.

As a further preferred embodiment, the organic solvent during salt formation in step 1) of the preparation method is selected from the group consisting of methanol, ethanol, *n*-propanol, isopropanol, dichloromethane, heptane, acetonitrile, acetone, methyl ethyl ketone, toluene, 1,4-dioxane, tetrahydrofuran, *N*,*N*-dimethylformamide, ethyl acetate, isopropyl acetate, methyl *tert*-butyl ether and 2-methoxyethyl ether, and mixtures thereof and aqueous solutions thereof.

A third aspect of the present invention provides a preparation method for the aforementioned crystalline basic salt of the compound of formula (I), comprising the following step: transforming one crystalline form of the basic salt of the compound of formula (I) into another crystalline form of the salt by using a crystalline form transformation method, wherein the crystalline form transformation method includes heating or suspension crystalline form transformation in a solvent.

As a preferred embodiment, the solvent is selected from the group consisting of methanol, ethanol, *n*-propanol, isopropanol, dichloromethane, heptane, acetonitrile, acetone, methyl ethyl ketone, toluene, 1,4-dioxane, tetrahydrofuran, *N*,*N*-dimethylformamide, ethyl acetate, isopropyl acetate, methyl *tert*-butyl ether and 2-methoxyethyl ether, and mixtures thereof and aqueous solutions thereof.

A fourth aspect of the present invention provides a crystalline form of the compound of formula (I) in free form:

(I)

As a preferred embodiment, the crystalline form of the compound of formula (I) in free form is crystalline form C, crystalline form D, crystalline form E or crystalline form F.

As a further preferred embodiment, the crystalline form of the compound of formula (I) in free form is a crystalline form C having an X-ray powder diffraction (XRPD) pattern comprising five or more peaks at angles of diffraction (2θ) of 7.80 ±0.2°, 11.97±0.2°, 12.42±0.2°, 15.98±0.2°, 17.37±0.2°, 18.82±0.2°, 22.47±0.2°, 23.30±0.2°, 25.01±0.2° and 25.63 ±0.2°.

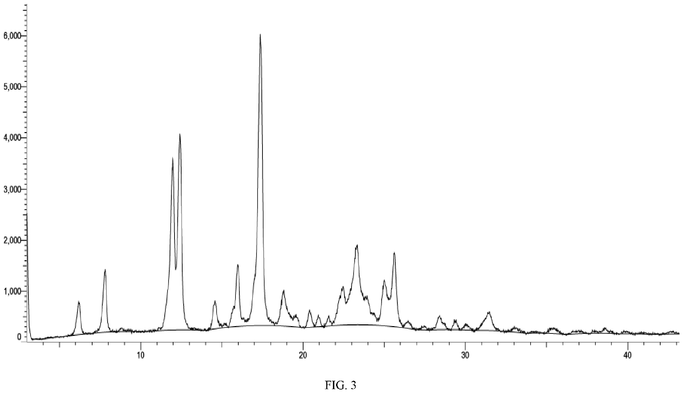

As a more further preferred embodiment, the X-ray powder diffraction (XRPD) pattern of the crystalline form C comprises peaks substantially identical (±0.2°) to those at angles of diffraction (20) shown in FIG. 3, and X-ray powder diffraction data thereof are shown in Table 3:

Table 3

| 2θ (°) | Intensity% | 2θ (°) | Intensity% |
|---|---|---|---|
| 6.21 | 10.9% | 24.35 | 3.6% |
| 7.80 | 21.1% | 25.01 | 15.6% |
| 8.82 | 1.1% | 25.63 | 25.5% |
| 11.97 | 59.2% | 26.48 | 2.6% |
| 12.42 | 67.2% | 27.48 | 1.3% |
| 14.59 | 9.5% | 28.41 | 4.7% |
| 15.17 | 1.4% | 28.58 | 2.4% |
| 15.81 | 8.7% | 29.38 | 2.9% |
| 15.98 | 21.2% | 30.09 | 2.0% |
| 17.37 | 100.0% | 31.47 | 6.3% |
| 18.82 | 12.1% | 33.05 | 1.1% |
| 19.52 | 4.0% | 35.36 | 1.9% |
| 20.39 | 6.0% | 38.00 | 1.0% |
| 20.94 | 3.9% | 38.63 | 1.8% |
| 21.60 | 3.5% | 39.90 | 1.0% |
| 22.47 | 13.3% | 42.72 | 0.8% |
| 23.30 | 27.1% | 43.95 | 0.7% |
| 23.86 | 9.6% | | |

The crystalline form is designated free-form crystalline form C and has a melting point of 169.5 °C.

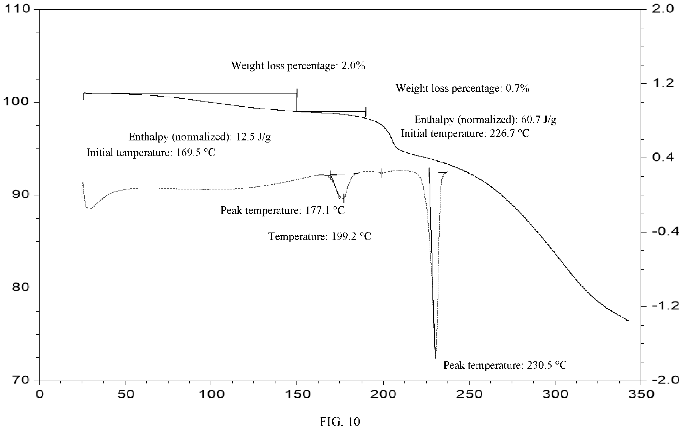

As the most preferred embodiment, the crystalline form of the compound of formula (I) is a free-form crystalline form C having DSC/TGA graphs that are substantially those shown in FIG. 10.

As a further preferred embodiment, the crystalline form of the compound of formula (I) in free form is a crystalline form D having an X-ray powder diffraction (XRPD) pattern comprising five or more peaks at angles of diffraction (20) of 7.97 ±0.2°, 11.82±0.2°, 12.18±0.2°, 14.87±0.2°, 16.13±0.2°, 17.18±0.2°, 19.21±0.2°, 20.72±0.2° and 25.28±0.2°.

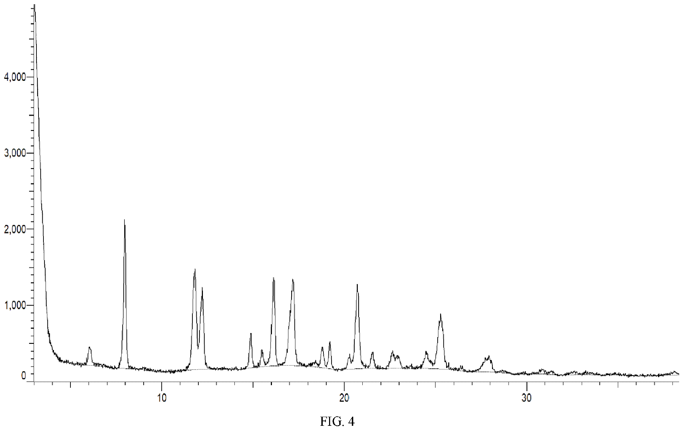

As a more further preferred embodiment, the X-ray powder diffraction (XRPD) pattern of the crystalline form D comprises peaks substantially identical (±0.2°) to those at angles of diffraction (20) shown in FIG. 4, and X-ray powder diffraction data thereof are shown in Table 4:

Table 4

| 2θ (°) | Intensity% | 2θ (°) | Intensity% |
|---|---|---|---|
| 6.06 | 12.4% | 18.81 | 14.5% |
| 7.97 | 100.0% | 19.21 | 19.4% |
| 11.82 | 66.7% | 20.72 | 58.4% |
| 12.18 | 50.6% | 21.55 | 11.7% |
| 14.87 | 22.9% | 24.48 | 13.2% |
| 15.48 | 13.6% | 25.28 | 37.9% |
| 16.13 | 61.3% | 27.88 | 10.9% |
| 17.18 | 60.5% | | |

The crystalline form is designated free-form crystalline form D and has a melting point of 239.9 °C.

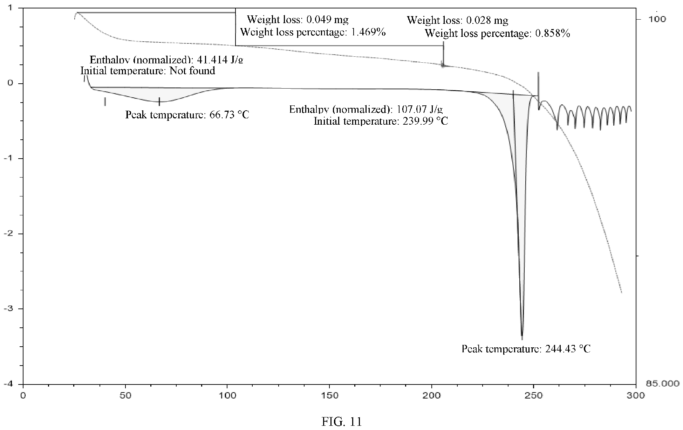

As the most preferred embodiment, the crystalline form of the compound of formula (I) is a free-form crystalline form D having DSC/TGA graphs that are substantially those shown in FIG. 11.

As a further preferred embodiment, the crystalline form of the compound of formula (I) in free form is a crystalline form E having an X-ray powder diffraction (XRPD) pattern comprising five or more peaks at angles of diffraction (20) of 8.16 ±0.2°, 12.05±0.2°, 12.83±0.2°, 15.19±0.2°, 16.18±0.2°, 16.59±0.2°, 17.62±0.2°, 18.41±0.2°, 22.90±0.2° and 25.65 ±0.2°.

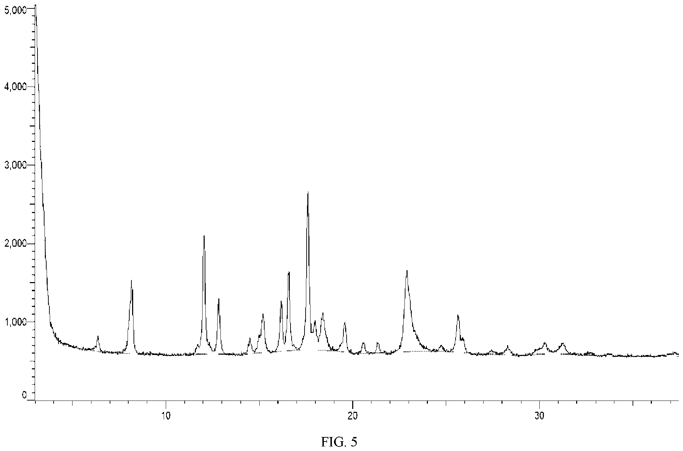

As a more further preferred embodiment, the X-ray powder diffraction (XRPD) pattern of the crystalline form E comprises peaks substantially identical (±0.2°) to those at angles of diffraction (2θ) shown in FIG. 5, and X-ray powder diffraction data thereof are shown in Table 5:

Table 5

| 2θ (°) | Intensity% | 2θ (°) | Intensity% |
|---|---|---|---|
| 6.37 | 9.4% | 17.62 | 100.0% |
| 8.16 | 46.1% | 18.41 | 25.3% |
| 12.05 | 74.6% | 19.59 | 18.8% |
| 12.83 | 36.5% | 20.57 | 6.6% |
| 14.50 | 11.2% | 21.33 | 7.7% |
| 15.19 | 24.7% | 22.90 | 50.8% |
| 16.18 | 32.6% | 25.65 | 24.7% |
| 16.59 | 47.8% | | |

The crystalline form is designated free-form crystalline form E and has a melting point of 226 °C.

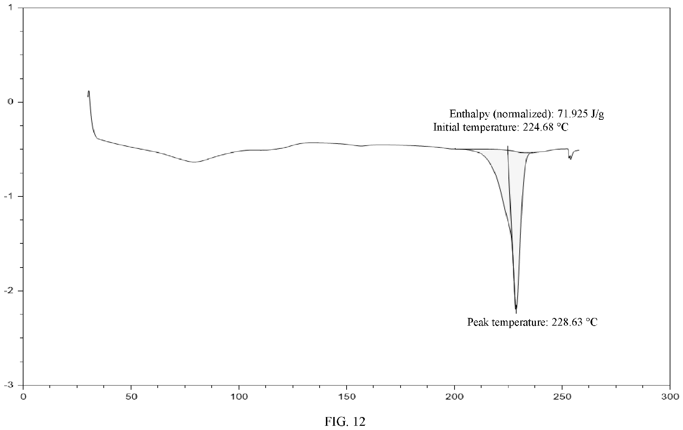

As the most preferred embodiment, the crystalline form of the compound of formula (I) is a free-form crystalline form E having a DSC graph that is substantially the one shown in FIG. 12.

As a further preferred embodiment, the crystalline form of the compound of formula (I) in free form is a crystalline form F having an X-ray powder diffraction (XRPD) pattern comprising five or more peaks at angles of diffraction (20) of 6.92 ±0.2°, 9.10±0.2°, 12.42±0.2°, 13.46±0.2°, 14.38±0.2°, 15.84±0.2°, 19.04±0.2°, 22.00±0.2° and 26.23±0.2°.

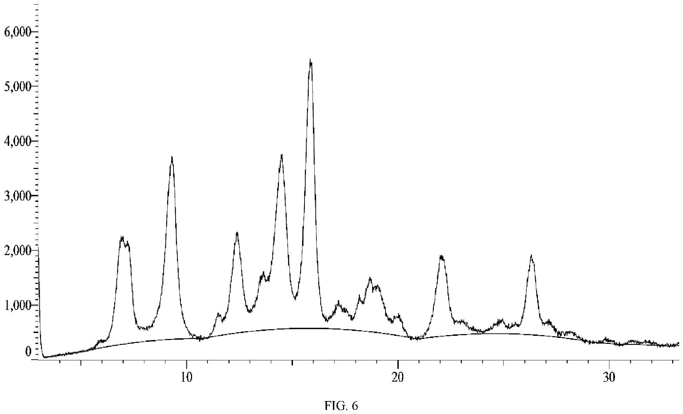

As a more further preferred embodiment, the X-ray powder diffraction (XRPD) pattern of the crystalline form F comprises peaks substantially identical (±0.2°) to those at angles of diffraction (20) shown in FIG. 6, and X-ray powder diffraction data thereof are shown in Table 6:

Table 6

| 2θ (°) | Intensity% | 2θ (°) | Intensity% |
|---|---|---|---|
| 5.79 | 10.7% | 14.38 | 42.9% |
| 6.92 | 40.6% | 15.84 | 59.3% |
| 9.10 | 100.0% | 19.04 | 11.8% |

(continued)

| 2θ (°) | Intensity% | 2θ (°) | Intensity% |
|---|---|---|---|
| 11.39 | 8.1% | 22.00 | 12.0% |
| 12.42 | 24.0% | 26.23 | 13.7% |
| 13.46 | 21.1% | | |

The crystalline form is designated free-form crystalline form F and has a melting point of 221 °C.

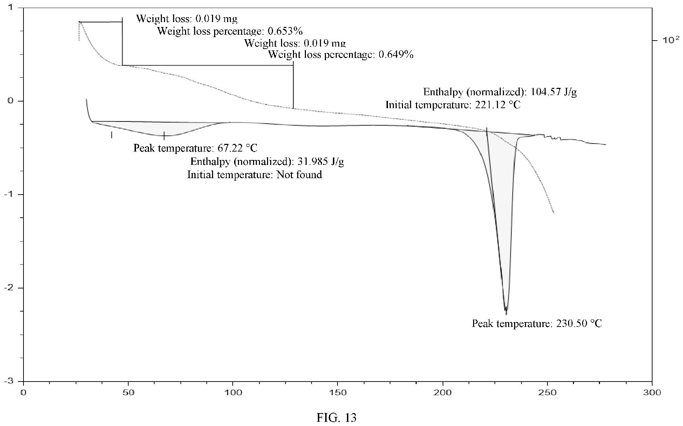

As the most preferred embodiment, the crystalline form of the compound of formula (I) is a free-form crystalline form F having DSC/TGA graphs that are substantially those shown in FIG. 13.

A fifth aspect of the present invention provides a use of the aforementioned crystalline form of the compound of formula (I) in free form, wherein the crystalline form of the compound of formula (I) in free form is used as a starting material to prepare the crystalline basic salt of the compound of formula (I) according to any one of the above.

A sixth aspect of the present invention provides a pharmaceutical composition comprising a clinically effective amount of the aforementioned crystalline basic salt of the compound of formula (I) or the crystalline form of the compound of formula (I) in free form and a pharmaceutically acceptable carrier.

As a preferred embodiment, the clinically effective amount means that the pharmaceutical composition comprises 0.01-99.0% W/W of the crystalline basic salt of the compound of formula (I) or the crystalline form of the compound of formula (I) in free form relative to a total content of the pharmaceutical composition.

A seventh aspect of the present invention provides a use of the aforementioned crystalline basic salt of the compound of formula (I) or the aforementioned crystalline form of the compound of formula (I) in free form in preparation of a medicament for treating a PD-1/PD-L1 signal pathway-mediated tumor, immunity-related disease and disorder, communicable disease, infectious disease or metabolic disease; preferably, the tumor is cancer.

As a preferred embodiment, the infectious disease is a bacterial infectious disease, a viral infectious disease or a fungal infectious disease.

As a preferred embodiment, the tumor is lymphoma, sarcoma, melanoma, glioblastoma, synovioma, meningioma, biliary tract tumor, neuroma, seminoma, nephroblastoma, hepatocellular papilloma, papilloma, leiomyoma, rhabdomyoma, hemangioma, lymphangioma, osteoma, lipoma, fibroma, central nervous system tumor, spinal axis tumor, brain stem glioma, multiple myeloma, ovarian tumor, myelodysplastic syndrome or mesothelioma, anal cancer, testicular cancer, urethral carcinoma, penile cancer, bladder cancer, ureteral cancer, uterine cancer, ovarian cancer, fallopian tube cancer, cervical cancer, vaginal cancer, vulvar cancer, Merkel cell carcinoma, embryonal carcinoma, chronic or acute leukemia, bronchial carcinoma, esophageal cancer, nasopharyngeal carcinoma, hepatocellular carcinoma, basal cell carcinoma, lung cancer, adenocarcinoma, papillary carcinoma, rectal cancer, colon cancer, gastric cancer, head and neck cancer, bone cancer, skin cancer, small intestine cancer, endocrine cancer, renal pelvic carcinoma, epidermoid carcinoma, transitional cell carcinoma or choriocarcinoma;

the immunity-related disease and disorder is rheumatic arthritis, renal failure, lupus erythematosus, asthma, psoriasis, ulcerative colitis, pancreatitis, allergy, fibrosis, anemia, fibromyalgia, Alzheimer's disease, congestive heart failure, stroke, aortic valve stenosis, arteriosclerosis, osteoporosis, Parkinson's disease, Crohn's disease, ulcerative colitis, allergic contact dermatitis and eczema, systemic sclerosis or multiple sclerosis;

the communicable disease or infectious disease is sepsis, liver infection, hepatitis A, hepatitis B, hepatitis C, hepatitis D, herpes virus, papillomavirus or influenza; and

the metabolic disease is diabetes, diabetic ketoacidosis, hyperglycemic hyperosmolar syndrome, hypoglycemia, gout, malnutrition, vitamin A deficiency, scurvy, vitamin D deficiency or osteoporosis.

As a preferred embodiment, the lymphoma is lymphocytic lymphoma, primary central nervous system lymphoma, T cell lymphoma, diffuse large B cell lymphoma, follicle center lymphoma, Hodgkin lymphoma, non-Hodgkin lymphoma or primary mediastinal large B cell lymphoma;

the sarcoma is Kaposi's sarcoma, fibrosarcoma, liposarcoma, chondrosarcoma, osteosarcoma, leiomyosarcoma, rhabdomyosarcoma, soft tissue sarcoma, angiosarcoma or lymphangiosarcoma; and

the chronic or acute leukemia is acute myeloid leukemia, chronic myeloid leukemia, acute lymphoblastic leukemia, chronic granulocytic leukemia or chronic lymphoblastic leukemia.

As a preferred embodiment, the tumor is small cell lung cancer, squamous non-small cell lung cancer, non-squamous non-small cell lung cancer, chondroma, colorectal cancer, gastrointestinal cancer, endometrial cancer, head and neck squamous cell carcinoma, abdominal wall carcinoma, renal cell carcinoma or recurrent or existing drug-resistant

prostate cancer.

As a preferred embodiment, the tumor is thymic tumor, pleomorphic adenoma, renal tubule adenoma, cystadenoma, pituitary adenoma, prostate cancer, thyroid cancer, parathyroid cancer, adrenal cancer, breast cancer, cystadenocarcinoma or pancreatic cancer.

An eighth aspect of the present invention provides the aforementioned crystalline basic salt of the compound of formula (I) or the aforementioned crystalline form of the compound of formula (I) in free form for use as a medicament for treating a PD-1/PD-L1 signal pathway-mediated tumor, immunity-related disease and disorder, communicable disease, infectious disease or metabolic disease.

The present invention also relates to a method for treating a PD-1/PD-L1 signal pathway-mediated tumor, immunity-related disease and disorder, communicable disease, infectious disease or metabolic disease, comprising administering to a patient in need thereof the aforementioned crystalline basic salt of the compound of formula (I) or the aforementioned crystalline form of the compound of formula (I) in free form.

## BRIEF DESCRIPTION OF THE DRAWINGS

FIG. 1 shows an X-ray powder diffraction pattern of the crystalline form A of disodium salt hydrate of the compound of formula (I) of the present invention. The abscissa represents the value of 2θ (degrees), and the ordinate represents peak intensity.

FIG. 2 shows an X-ray powder diffraction pattern of the crystalline form B of disodium salt anhydrate of the compound of formula (I) of the present invention. The abscissa represents the value of 2θ (degrees), and the ordinate represents peak intensity.

FIG. 3 shows an X-ray powder diffraction pattern of the free-form crystalline form C of the compound of formula (I) of the present invention. The abscissa represents the value of 2θ (degrees), and the ordinate represents peak intensity.

FIG. 4 shows an X-ray powder diffraction pattern of the free-form crystalline form D of the compound of formula (I) of the present invention. The abscissa represents the value of 2θ (degrees), and the ordinate represents peak intensity.

FIG. 5 shows an X-ray powder diffraction pattern of the free-form crystalline form E of the compound of formula (I) of the present invention. The abscissa represents the value of 2θ (degrees), and the ordinate represents peak intensity.

FIG. 6 shows an X-ray powder diffraction pattern of the free-form crystalline form F of the compound of formula (I) of the present invention. The abscissa represents the value of 2θ (degrees), and the ordinate represents peak intensity.

FIG. 7 shows the structure of a unit cell of a single crystal of the crystalline form A of disodium salt hydrate of the compound of formula (I) of the present invention.

FIG. 8 shows DSC/TGA graphs of the crystalline form A of disodium salt hydrate of the compound of formula (I) of the present invention. The abscissa represents temperature (°C), the left ordinate represents heat flow (w/g), and the right ordinate represents weight (%).

FIG. 9 shows DSC/TGA graphs of the crystalline form B of disodium salt anhydrate of the compound of formula (I) of the present invention. The abscissa represents temperature (°C), the left ordinate represents heat flow (w/g), and the right ordinate represents weight (%).

FIG. 10 shows DSC/TGA graphs of the free-form crystalline form C of the compound of formula (I) of the present invention. The abscissa represents temperature (°C), the left ordinate represents weight (%), and the right ordinate represents heat flow (w/g).

FIG. 11 shows DSC/TGA graphs of the free-form crystalline form D of the compound of formula (I) of the present invention. The abscissa represents temperature (°C), the left ordinate represents heat flow (w/g), and the right ordinate represents weight (%).

FIG. 12 shows a DSC graph of the free-form crystalline form E of the compound of formula (I) of the present invention. The abscissa represents temperature (°C), and the ordinate represents heat flow (w/g).

FIG. 13 shows DSC/TGA graphs of the free-form crystalline form F of the compound of formula (I) of the present invention. The abscissa represents temperature (°C), the left ordinate represents heat flow (w/g), and the right ordinate represents weight (%).

FIG. 14 shows a DVS graph of the free-form crystalline form C of the compound of formula (I) of the present invention. The abscissa represents relative humidity (%), and the ordinate represents weight change (%).

FIG. 15 shows a DVS graph of the free-form crystalline form D of the compound of formula (I) of the present invention. The abscissa represents relative humidity (%), and the ordinate represents weight change (%).

FIG. 16 shows a DVS graph of the free-form crystalline form F of the compound of formula (I) of the present invention. The abscissa represents relative humidity (%), and the ordinate represents weight change (%).

FIG. 17 shows a DVS graph of the crystalline form A of disodium salt hydrate of the compound of formula (I) of the present invention. The abscissa represents relative humidity (%), and the ordinate represents weight change (%).

FIG. 18 shows an X-ray powder diffraction pattern of an amorphous potassium salt of the compound of formula (I) of the present invention. The abscissa represents the value of 2θ (degrees), and the ordinate represents peak intensity.

FIG. 19 shows an X-ray powder diffraction pattern of an amorphous arginine salt of the compound of formula (I) of the present invention. The abscissa represents the value of 2θ (degrees), and the ordinate represents peak intensity.

## DETAILED DESCRIPTION OF THE INVENTION

The inventors of the present invention have intensively studied different aggregate forms of the compound of formula (I) and provide a crystalline free form of the PD-1/PD-L1 inhibitor or a basic salt thereof. The crystalline free form of the compound greatly improves physicochemical properties, such as hygroscopicity, solubility and physicochemical stability, of the compound of formula (I), and the crystalline basic salt of the compound greatly improves physicochemical properties such as solubility and physicochemical stability, so that the crystalline free form of the compound of formula (I) or the basic salt thereof can satisfy the need to develop clinical pharmaceutical formulations, has very important clinical application value, can be widely applied to the preparation of drugs for treating tumors, immunity-related diseases and disorders, communicable diseases, infectious diseases or metabolic diseases, particularly drugs for treating ovarian cancer, pancreatic cancer, prostate cancer, breast cancer, cervical cancer, glioblastoma, multiple myeloma, metabolic diseases, neurodegenerative diseases, primary tumor metastasis or metastatic cancer in bones, and is expected to be developed into a new-generation PD-1/PD-L1 inhibitor drug soon. The present invention is achieved on this basis.

Detailed description: Unless otherwise stated, the following terms used in the specification and claims have the following meanings.

"Pharmaceutical composition" refers to a mixture containing one or more of the compounds described herein or a physiologically/pharmaceutically acceptable salt or pro-drug thereof and other chemical components, as well as other components such as physiologically/pharmaceutically acceptable carriers and excipients. The purpose of the pharmaceutical composition is to promote administration to an organism and facilitate the absorption of the active ingredient, so that the active ingredient can exert biological activity.

The compound of formula (I) has a variety of separated free forms or basic salts, showing polymorphism or monomorphism. For example, each of the sodium salts is shown as a polymorph. These "polymorphs" differ in terms of their X-ray powder diffraction patterns, physicochemical and pharmacokinetic properties and thermodynamic stability.

As used herein, "salt" refers to a compound prepared by reacting an organic acid or base medicament with a pharmaceutically acceptable inorganic or organic acid or base.

### Methods and Materials

Diffraction data of the crystalline free form of the compound of formula (I) or the basic salt thereof were collected at room temperature using a Bruker D8 ADVANCE (Bruker, Germany) X-ray powder diffractometer with an X-ray source of Cu Kα (λ = 1.5406 Å, light tube voltage, current: 40 kV, 40 mA). When a sample was prepared, an appropriate amount of the sample was placed on a zero-background sample tray, and the surface was flattened by gently pressing. The scan range for the sample was from 3° to 40° (20), the step size was 0.02° (2θ), and the duration of each step of scanning was 0.02 s. Diffraction patterns were analyzed using DIFFRAC.EVA (V4.3.1). Measurement differences associated with such X-ray powder diffraction analyses result from a variety of factors including: (a) errors in sample preparation (e.g., sample height), (b) instrument errors, (c) calibration errors, (d) operator errors (including those errors present when determining peak positions), and (e) the nature of the material (e.g., preferred orientation errors). Calibration errors and sample height errors often result in a shift of all the peaks in the same direction. In general, this calibration factor will cause the measured peak positions to be in agreement with the expected peak positions and may be in the range of the expected 2θ value ± 0.2°. The angle 2θ values (°) and intensity values (as a % of the value of the highest peak) for each polymorph obtained in the examples of the present invention are shown in Tables 1-6.

The experimental method of characterizing the crystalline free form of the compound of formula (I) or the basic salt thereof using differential scanning calorimetry (DSC) is as follows: a small amount of the crystalline sample was placed in an aluminum pan that was compatible with the instrument and could be covered with a lid; after the sample was loaded, the aluminum pan was covered with a lid and then placed into the instrument for analysis. In this patent, all the instruments used for differential scanning calorimetry were TA Discovery DSC25, the scanning parameter was set to using a nitrogen atmosphere, and the warming rate was 10 °C/min.

The experimental method of characterizing the crystalline free form of the compound of formula (I) or the basic salt thereof using thermogravimetric analysis (TGA) is as follows: a small amount of the crystalline sample was placed in an aluminum pan that was compatible with the instrument; after the sample was loaded, the aluminum pan was placed into the instrument for analysis. In this patent, all the instruments used for thermogravimetric analysis were TA Discovery TGA550, the scanning parameter was set to using a nitrogen atmosphere, and the warming rate was 10 °C/min.

The experimental method of characterizing the crystalline free form of the compound of formula (I) or the basic

salt thereof using dynamic vapor sorption (DVS) is as follows: a small amount of the crystalline sample was placed in a precision sample tray that was compatible with the instrument; after the sample was loaded, the sample tray was placed into the instrument for analysis. In this patent, the instruments used for dynamic vapor sorption were all the DVS Intrinsic plus model, and the following experimental parameters were set: nitrogen was used as the carrier gas, a constant temperature of 25 °C was set, the rate of mass percentage change over a unit of time (dm/dt) = 0.002%/min was used as a criterion for determining if equilibrium is reached, and the program humidity change cycle was set to 50-95-0-95-50% RH; within the range of 0-90% RH, a change of 10% RH was taken as a step, and within the range of 90% RH-95% RH, a change of 5% RH was taken as a step.

The stability and solubility of the crystalline free form of the compound of formula (I) or the basic salt thereof of the present application were determined by HPLC. The specific test method is shown in the table below.

| **Instrument** | Thermo HPLC | |
|---|---|---|
| **Chromatographic column** | Xbridge-C18 | |
| **Chromatographic column supplier** | Waters | |
| **Particle size (μm)** | 3.5 | |
| **Size (mm)** | 4.6 × 150 | |
| **Column temperature (°C)** | 40 | |
| **Flow rate (mL/minute)** | 0.8 | |
| **Injection volume (μL)** | 10 | |
| **Dilution solvent** | MeOH | |
| **Sample concentration (μg/mL)** | 400 | |
| **Wavelength (nm)** | 277 | |
| **Mobile phase A** | 0.1% TFA in water | |
| **Mobile phase B** | 0.1% TFA in acetonitrile | |
| **Run time (minutes)** | 21.0 | |
| **Elution gradient** | % B | Minutes |
| | 5 | Initial |
| | 95 | 19 |
| | 95 | 20 |
| | 5 | 20.1 |
| | 5 | 21 |

The reagents in the examples of the present invention are known and commercially available, or can be synthesized using or according to methods known in the art. The starting material used in the study was prepared according to Patent No. WO2019149183A1, or was optionally subjected to conventional treatment in the art to obtain a free form.

Unless otherwise stated, all reactions of the present invention are carried out in a dry nitrogen or argon atmosphere with continuous magnetic stirring, the solvent is a dry solvent, and the temperature is in degrees centigrade (°C).

Unless otherwise specified, each of the various crystalline forms referred to in the present invention may be an anhydrous crystalline form or a hydrated crystalline form. For example, it is a hydrated crystalline form; preferably, each molecule of the crystal contains 1, 2, 3, 4 or 5 waters of crystallization; and more preferably, each molecule of the crystal contains 2 or 4 waters of crystallization.

**The present invention is further explained in detail below using the drawings and specific examples, which are illustrative only of particular embodiments of the present invention and should not be construed as limiting the scope of the present invention in any way.**

**Preparation of Specific Examples**

**Example 1. Preparation of Crystalline Form A of Disodium Salt Hydrate**

About 10 mg of the compound of formula (I) in free form was dissolved in 0.5 mL of ethanol/water (volume ratio: 10:1), and a solution of 2 equivalents of sodium hydroxide in ethanol/water was added. The mixture was stirred at room temperature for 3 days and filtered, and the filter cake was dried in a drying oven at 50 °C. The crystalline form A of disodium salt hydrate was a solid with high crystallinity and had a solid-solid crystalline form transformation peak at 237.3 °C.

Analysis showed that the crystalline form had the XRPD pattern shown in FIG. 1 and the DSC/TGA graphs shown in FIG. 8.

**Example 2. Preparation of Crystalline Form B of Disodium Salt Anhydrate**

About 10 mg of the crystalline form A of disodium salt hydrate of the compound of formula (I) was heated to 245 °C to obtain the crystalline form B of disodium salt anhydrate, which had a melting point of 316.4 °C. The crystalline form B of disodium salt anhydrate was a solid with high crystallinity.

Analysis showed that the crystalline form had the XRPD pattern shown in FIG. 2 and the DSC/TGA graphs shown in FIG. 9.

**Example 3. Preparation of Free-Form Crystalline Form C**

About 25 mg of the compound of formula (I) in free form was added to 200 μL of ethyl acetate and suspended at 45 °C for 7 days. A solid sample was then isolated by centrifugation and dried under vacuum at 40 °C overnight. The free-form crystalline form C was a solid with high crystallinity and had a melting peak at 169.5 °C.

Analysis showed that the crystalline form had the XRPD pattern shown in FIG. 3 and the DSC/TGA graphs shown in FIG. 10.

**Example 4. Preparation of Free-Form Crystalline Form D**

About 10 mg of the compound of formula (I) in free form was added to 200 μL of ethyl acetate and suspended at 45 °C for 7 days. The mixture was filtered, and the filter cake was dried in a drying oven at 50 °C. The free-form crystalline form D was a solid with moderate crystallinity, and DSC showed a melting peak at 239.9 °C.

Analysis showed that the crystalline form had the XRPD pattern shown in FIG. 4 and the DSC and TGA graphs shown in FIG. 11.

**Example 5. Preparation of Free-Form Crystalline Form E**

5 mg of the free-form crystalline form C of the compound of formula (I) was heated to 166 °C to obtain a solid sample of the free-form crystalline form E. The free-form crystalline form E was a solid with moderate crystallinity and had a melting peak at 224.7 °C.

Analysis showed that the crystalline form had the XRPD pattern shown in FIG. 5 and the DSC graph shown in FIG. 12.

**Example 6. Preparation of Free-Form Crystalline Form F**

5 mg of the compound of formula (I) in free form was added to 200 μL of methanol and suspended at 50 °C for 7 days. Then, a solid sample was separated by centrifugation and dried under vacuum at 40 °C overnight. The free-form crystalline form F was a solid with moderate crystallinity and had a melting peak at 221.1 °C.

Analysis showed that the crystalline form had the XRPD pattern shown in FIG. 6 and the DSC and TGA graphs shown in FIG. 13.

**Example 7. Preparation of Amorphous Potassium Salt**

About 10 mg of the compound of formula (I) in free form was dissolved in 0.5 mL of trifluoroethanol, and a solution of 2 equivalents of potassium hydroxide in trifluoroethanol was added. The mixture was stirred at room temperature for 3 days and filtered, and the filter cake was dried in a drying oven at 50 °C.

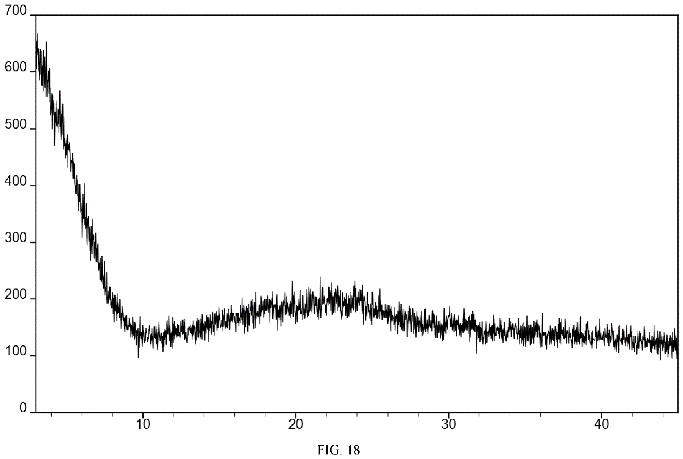

Analysis showed that the product had the XRPD pattern shown in FIG. 18. The potassium salt obtained was an amorphous solid.

### Example 8. Preparation of Amorphous Arginine Salt

About 10 mg of the compound of formula (I) in free form was dissolved in 0.5 mL of trifluoroethanol, and a solution of 2 equivalents of arginine in trifluoroethanol was added. The mixture was stirred at room temperature for 3 days and filtered, and the filter cake was dried in a drying oven at 50 °C.

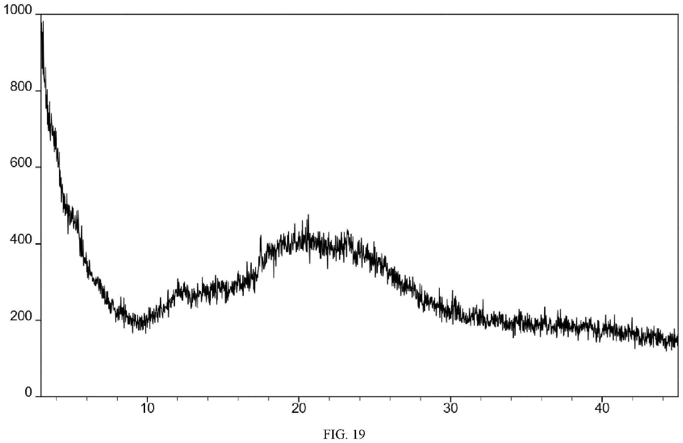

Analysis showed that the product had the XRPD pattern shown in FIG. 19. The arginine salt obtained was an amorphous solid.

### Example 9. Single Crystal Structure Analysis of Crystalline Form A of Disodium Salt Hydrate

#### 1. Experimental instruments

Transmission electron microscope: Thermo Scientific Glacios
Detector: Thermo Scientific Ceta-D
Sample rod: Autoloader

#### 2. Experimental conditions

Voltage: 200 kV
Temperature: 83 K
Vacuum value: $10^{-7}$ Pa high vacuum

#### 3. Application software

Data collection: Thermo Scientific EPU-D
Data processing: XDS, SHELXT and SHELXL

#### 4. Sample preparation

A small amount of the powdered sample was directly transferred onto a TEM grid, and the excess powder was blown away with a rubber bulb. The grid was rapidly plunged into liquid ethane using a Thermo Scientific Vitrobot cryo-electron microscope sample preparation system. After being frozen, the sample was transferred to liquid nitrogen for storage and then loaded into the electron microscope for observation.

#### 5. Image collection

Multiple particles of appropriate size with clear diffraction signals were selected, and a series of diffraction data generated by rotation of the particles were collected automatically using EPU-D software.

#### 6. Data processing

The diffraction images were indexed and subjected to intensity integration to obtain unit cell parameters and HKL files. Multiple sets of data were merged to solve the crystal structure.

#### 7. Analysis results

From 26 different particles of the sodium salt of the compound of formula (I), 19 sets of diffraction images were collected. These images were each indexed and integrated using XDS, and the following unit cell constants were obtained: a = 20.00(5) Å, b = 5.798(16) Å, c = 20.44(11) Å, $\alpha$ = 90°, $\beta$ = 94.8(2)°, $\gamma$ = 90° and unit cell volume V = 2362(15) $Å^3$.

**8. Structure determination**

The 9 best-quality sets of data were merged using XSCALE. The total data set contained a total of 16,135 diffraction points, of which 2821 were independent diffraction points. From the above data, the crystal structure of the crystalline form A of disodium salt hydrate was successfully determined. It is a monoclinic crystal system with a space group of C2 (No. 5), a molecular weight of 450.48 g·mol$^{-1}$ and Z' = 0.5. The asymmetric unit consists of 0.5 API anions, 1 sodium ion and 2 water molecules. The structure of a unit cell of the single crystal is shown in FIG. 7. Crystallographic data and refinement parameters are shown in the table below.

| **Chemical formula** | $C_{46}H_{50}N_6O_6^{-} \cdot 2Na^{+} \cdot 4H_2O$ |
|---|---|
| **Molecular weight** | 900.97 g·mol$^{-1}$ |
| **Temperature** | 83(2) K |
| **Wavelength** | 0.02508 Å |
| **Crystal system, space group** | Monoclinic, C2 (No. 5) |
| **Unit cell parameters** | a = 20.00(5) Å<br>b = 5.798(16) Å<br>c = 20.44(11) Å<br>$\alpha$ = 90°<br>$\beta$ = 94.8(2)°<br>$\gamma$ = 90° |
| **Volume** | 2362(15) Å$^3$ |
| **Z'** | 0.5 |
| **Density** | 1.267 g/cm$^3$ |
| **Resolution** | 0.90 Å |
| **All diffraction points** | 16135 |
| **Independent diffraction points** | 2821 |
| **Completeness** | 82.7 % |
| **$R_{int}$** | 0.2522 |
| **Initial model generation method** | *ab initio* |
| **Hydrogen atom refinement** | Geometric constraints |
| **Goodness-of-fit on $F^2$** | 1.284 |
| **$R_1$ [I ≥ 2sigma(I)]** | 0.1534 |
| **$R_1$ [all data]** | 0.1736 |
| **$wR_2$[I ≥ 2sigma(I)]** | 0.3589 |
| **$wR_2$[all data]** | 0.3752 |

**Example 10. Solubility Determination**

The desired amount of the compound was weighed into a glass vial, and the desired amount of a vehicle was added. A stir bar was added to the vial, and the mixture was stirred at room temperature for 24 hrs. After 24 hrs, the appearance of the sample was observed. An appropriate amount of the sample was filtered through a PVDF 0.45 μm filter membrane, and the filtrate was collected. The pH value of the filtrate was determined, and the sample was diluted with MeOH. The diluted sample was injected into an HPLC system to determine the concentration.

The process of preparing pH buffers is shown in the table below:

| pH value | Preparation process |
|---|---|
| 1.2 | 8.5 mL of a 0.2 M hydrochloric acid solution and 5 mL of a 0.2 M potassium chloride solution were added to a 20 mL volumetric flask, and the mixture was brought to volume with water and well mixed. The pH value was determined, and the pH should be 1.2±0.03. The pH was adjusted to 1.2 with hydrochloric acid or sodium hydroxide if necessary. |
| 4.7 | 359 mg of sodium acetate trihydrate solid was weighed into a 100 mL volumetric flask, and an appropriate amount of water was added to dissolve the solid. Then, 1.18 mL of a 2 N acetic acid solution was transferred into the volumetric flask, and the mixture was brought to volume with water and well mixed. The pH value was determined, and the pH should be 4.7±0.03. |
| 6.8 | 169.96 mg of anhydrous potassium dihydrogen phosphate and 29.17 mg of sodium hydroxide were added to a 25 mL volumetric flask, and the solution was brought to volume with purified water. The pH was adjusted to 6.8 with phosphoric acid. |

The process of preparing biological media is shown in the table below:

| Biological medium | Preparation process |
|---|---|
| FaSSIF fasted-state simulated intestinal fluid | 1.0471 g of concentrated FaSSIF solution was weighed into a 25 mL volumetric flask and diluted with an appropriate amount of purified water, and 55.9 mg of FaSSIF/FeSSIF/FaSSGF powder was added. The mixture was brought to volume (25 mL) and well mixed (pH 6.5). |
| FeSSIF fed-state simulated intestinal fluid | 2.0365 g of concentrated FeSSIF solution was weighed into a 25 mL volumetric flask and diluted with an appropriate amount of purified water, and 280.2 mg of FaSSIF/FeSSIF/FaSSGF powder was added. The mixture was brought to volume (25 mL) and well mixed (pH 5.0). |
| FaSSGF simulated gastric fluid | 3.6790 g of concentrated FaSSGF solution was weighed into a 100 mL volumetric flask and diluted with an appropriate amount of purified water, and 6.0 mg of FaSSIF/FeSSIF/FaSSGF powder was added. The mixture was brought to volume (100 mL) and well mixed (pH 1.6). |
| Note | The concentrated FaSSIF solution, concentrated FeSSIF solution and concentrated FaSSGF solution and FaSSIF/FeSSIF/FaSSGF powders used are all commercially available products (supplier: Biorelevant). |

The experimental results are shown below:

**Equilibrium solubility data of free-form crystalline forms C, D and F**

| No. | Vehicle | Target concentration (μg/mL) | Equilibrium solubility (μg/mL, $pH_{24\ hrs}$) | | |
|---|---|---|---|---|---|
| | | | Solubility of crystalline form C (μg/mL, $pH_{24\ hrs}$) | Solubility of crystalline form D (μg/mL, $pH_{24\ hrs}$) | Solubility of crystalline form F (μg/mL, $pH_{24\ hrs}$) |
| **1** | Water | 2000 | 3.84(6.78) | 51.93(5.82) | 69.47(5.52) |
| **2** | pH 1.2, HCl 100 mM | | 18.92(1.30) | 281.84(1.16) | 244.49(1.15) |
| **3** | pH 4.7, 50 mM acetic acid buffer | | 3.3(4.77) | 47.41(4.73) | 67.33(4.68) |
| **4** | pH6.8, 50 mM phosphoric acid buffer | | 2.31(6.89) | 42.11(6.90) | 61.01(6.93) |
| **5** | FaSSGF | | 8.9(1.56) | 87.01(1.57) | 117.15(1.58) |

(continued)

| No. | Vehicle | Target concentration (μg/mL) | Equilibrium solubility (μg/mL, $pH_{24\ hrs}$) | | |
|---|---|---|---|---|---|
| | | | Solubility of crystalline form C (μg/mL, $pH_{24\ hrs}$) | Solubility of crystalline form D (μg/mL, $pH_{24\ hrs}$) | Solubility of crystalline form F (μg/mL, $pH_{24\ hrs}$) |
| 6 | FaSSIF | | 3.21(6.41) | 78.44(6.43) | 306.56(6.42) |
| 7 | FeSSIF | | 5.34(4.90) | 433.75(4.94) | 1723.16(4.99) |

As can be seen from the above data, the free-form crystalline form C exhibited improved solubility in the buffer system having a pH of 1.2 compared to the other test conditions. Compared to the free-form crystalline form C, the free-form crystalline form D and free-form crystalline form F had solubility values that were increased by a factor of at least 10 or more under various test conditions, such as buffer systems having a pH of 1.2-6.8, water and simulated biological media (FaSSGF, FaSSIF and FeSSIF), indicating significantly improved solubility. More surprisingly, compared to the free-form crystalline form C, the free-form crystalline form F had a solubility value that was increased by a factor of nearly one hundred in the simulated biological medium FaSSIF and a solubility value that was increased by a factor of several hundreds in the simulated biological medium FeSSIF.

**Solubility data of crystalline form A of disodium salt hydrate and crystalline form B of disodium salt anhydrate**

| No. | Solvent/medium | Target concentration (μg/mL) | Equilibrium solubility (μg/mL, $pH_{24\ hrs}$) | |
|---|---|---|---|---|
| | | | Solubility of crystalline form B (μg/mL, $pH_{24\ hrs}$) | Solubility of crystalline form A (μg/mL, $pH_{24\ hrs}$) |
| 1 | Water | 2000 | ≥2000(9.96) | >2000(9.61) |
| 2 | pH 1.2, HCl 100 mM | | ≥2000(0.95) | 1120(1.01) |
| 3 | pH 4.7, 50 mM acetic acid buffer | | 135(4.92) | 25(4.61) |
| 4 | pH 6.8, 50 mM phosphoric acid buffer | | 194(7.03) | 34(6.88) |
| 5 | FaSSGF | | 485(1.72) | 22(1.73) |
| 6 | FaSSIF | | 474(6.93) | 303(6.93) |
| 7 | FeSSIF | | ≥2000(6.90) | >2000(7.95) |

As can be seen from the above data, the crystalline sodium salts of the compound of formula (I) exhibited significantly improved solubility, which was at least several tens of times higher than that of the free-form crystalline form C of the compound of formula (I). In particular, the crystalline form B of disodium salt anhydrate exhibited excellent solubility in various buffers and biological medium solutions.

**Example 11. Stability Determination**

An appropriate amount of the sample was placed in a vial and left to stand at 50 °C, 80 °C or 50 °C & 75% RH (open) for 7 days to evaluate thermal stability. An appropriate amount of the sample was placed in a transparent vial, an amber vial or an aluminum foil transparent vial and left to stand in a light box for 10 days to obtain a photostability evaluation under conditions of overall near-ultraviolet energy of 1.2 * $10^6$ Lux·hr and no less than 200 W·hr/m2. The experimental results are shown below.

**Stability data of free-form crystalline form C**

| Condition | Time | Appearance | Purity (%) | XRPD |
|---|---|---|---|---|
| **Initial** | 0 days | White powder | 98.42 | Free-form crystalline form C |
| **50 °C/75%RH** | 7 days | White powder | 98.43 | Free-form crystalline form C |
| **50 °C** | 7 days | White powder | 98.31 | Free-form crystalline form C |
| **80 °C** | 7 days | White powder | 97.90 | Free-form crystalline form C |
| **Transparent light exposure** | 9.2 days | White powder | 97.91 | Free-form crystalline form C |
| **Aluminum foil light exposure control** | 9.2 days | White powder | 98.51 | Free-form crystalline form C |

**Stability data of free-form crystalline form D**

| Condition | Time | Appearance | Purity (%) | XRPD |
|---|---|---|---|---|
| **Initial** | 0 days | White powder | 98.17 | Free-form crystalline form D |
| **50 °C/75%RH** | 7 days | White powder | 98.24 | Free-form crystalline form D |
| **50 °C** | 7 days | White powder | 98.13 | Free-form crystalline form D |
| **80 °C** | 7 days | White powder | 97.22 | Free-form crystalline form D |
| **Transparent light exposure** | 9.2 days | White powder | 96.06 | Free-form crystalline form D |
| **Aluminum foil light exposure control** | 9.2 days | White powder | 98.26 | Free-form crystalline form D |

**Stability data of free-form crystalline form F**

| Condition | Time | Appearance | Purity (%) | XRPD |
|---|---|---|---|---|
| Initial | 0 days | White powder | 99.44 | Free-form crystalline form F |
| 50 °C/75%RH | 11 days | White powder | 99.36 | Free-form crystalline form F |
| 50 °C | 11 days | White powder | 99.36 | Free-form crystalline form F |
| 80 °C | 11 days | White powder | 99.22 | Free-form crystalline form F |
| Transparent light exposure | 9.2 days | White powder | 99.43 | Free-form crystalline form F |
| Aluminum foil light exposure control | 9.2 days | White powder | 99.39 | Free-form crystalline form F |

**Stability data of crystalline form A of disodium salt hydrate**

| Condition | Time | Appearance | Purity (%) | XRPD |
|---|---|---|---|---|
| **Initial** | 0 days | White powder | 99.38 | Crystalline form A of disodium salt |
| **50 °C** | 7 days | White powder | 99.45 | Crystalline form A of disodium salt |
| **80 °C** | 7 days | White powder | 99.34 | Crystalline forms A + B of disodium salt |
| **50 °C/75%RH** | 7 days | White powder | 99.46 | Crystalline form A of disodium salt |
| **Transparent light exposure** | 9.2 days | White powder | 99.22 | Crystalline forms A + B of disodium salt |
| **Brown vial light exposure control** | 9.2 days | White powder | 99.50 | Crystalline form A of disodium salt |

(continued)

| Condition | Time | Appearance | Purity (%) | XRPD |
|---|---|---|---|---|
| **Aluminum foil light exposure control** | 9.2 days | White powder | 99.51 | Crystalline form A of disodium salt |

**Stability data of crystalline form B of disodium salt anhydrate**

| Condition | Time | Appearance | Purity (%) | XRPD |
|---|---|---|---|---|
| **Initial** | 0 days | White powder | 91.23 | Crystalline form B of disodium salt |
| **50 °C** | 7 days | White powder | 92.11 | Crystalline form B of disodium salt |
| **80 °C** | 7 days | White powder | 90.77 | Crystalline form B of disodium salt |
| **50 °C/75%RH** | 7 days | White powder | 92.97 | Crystalline form B of disodium salt |
| **Transparent light exposure** | 9.2 days | White powder | 83.10 | Crystalline form B of disodium salt |
| **Brown vial light exposure control** | 9.2 days | White powder | 88.10 | Crystalline form B of disodium salt |
| **Aluminum foil light exposure control** | 9.2 days | White powder | 89.50 | Crystalline form B of disodium salt |

As can be seen from the above results,

1) Under conditions of 50 °C, 50 °C/75% RH (open), 80 °C for 7 days and photostress for 10 days and overall near-ultraviolet energy of 1.2 * $10^6$ Lux·hr and no less than 200 W·hr, the free-form crystalline form C, crystalline form D and crystalline form F all exhibited extremely high physical and chemical stability.
2) The crystalline form A of disodium salt hydrate exhibited good chemical stability under various stability testing conditions, with no significant increase in impurity content; however, the crystalline form underwent crystalline form transformation under high temperature and light exposure. The crystalline form B of disodium salt anhydrate exhibited good physical stability under the stability testing conditions, and no crystalline form transformation was observed; however, the crystalline form was sensitive to light exposure. Therefore, the development of the crystalline forms of disodium salt requires attention to storage conditions, and high temperatures and exposure to light should be avoided.

**Example 12. Hygroscopic Behavior Test**

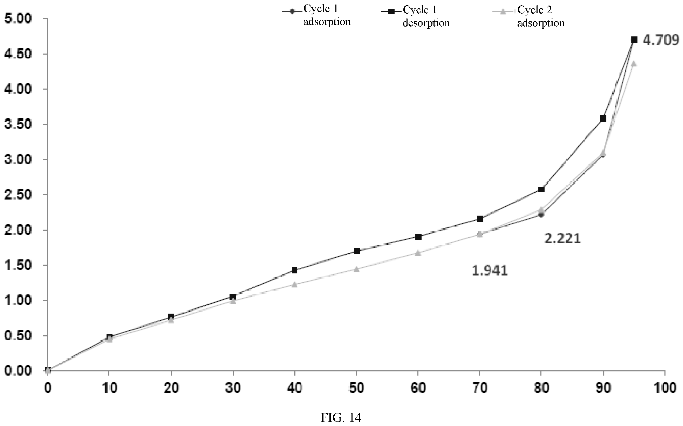

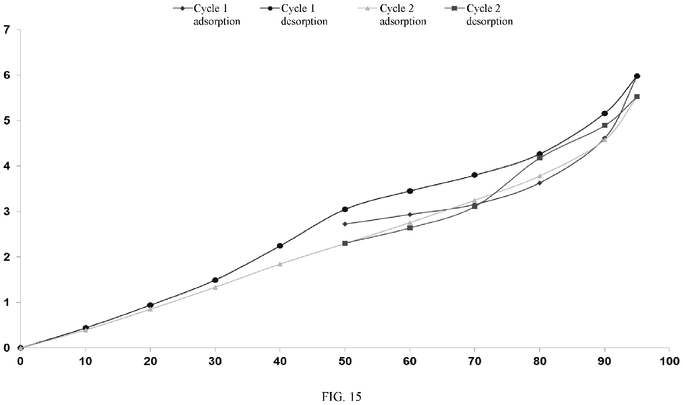

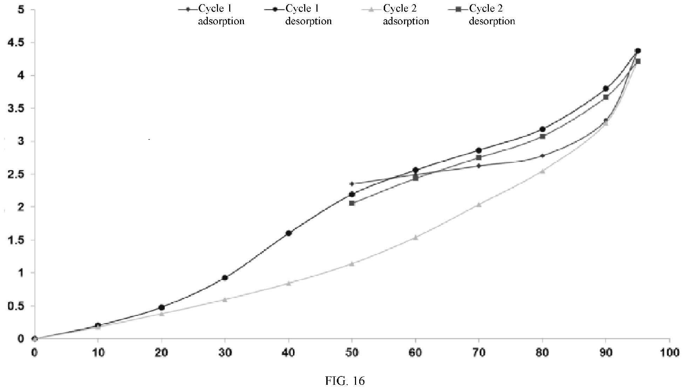

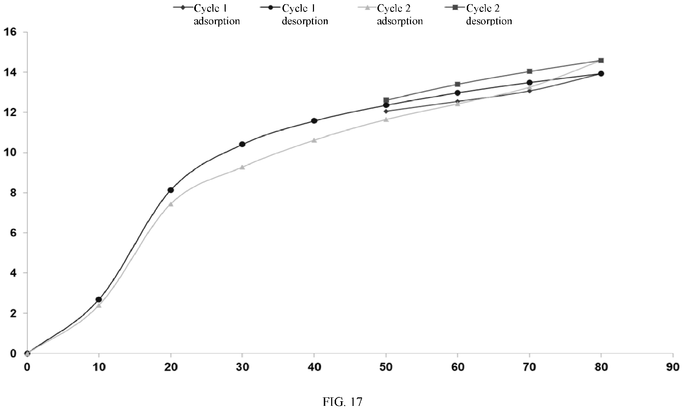

In the present invention, the hygroscopic weight increases (hygroscopic weight increase/weight before hygroscopy * 100%) of various crystalline forms under various relative humidity conditions were determined using the dynamic vapor sorption method, and the hygroscopicity of different crystalline compounds was evaluated. The results are shown in FIGs. 14-17 and the table below:

| Hygroscopic weight increase (%) / Crystalline form | Relative humidity (%) at 25 °C | | | | | | | | | | |
|---|---|---|---|---|---|---|---|---|---|---|---|
| | 0.0 | 10.0 | 20.0 | 30.0 | 40.0 | 50.0 | 60.0 | 65.0 | 70.0 | 80.0 | 90.0 |
| Free-form crystalline form C | 0.00 | 0.50 | 0.75 | 0.80 | 1.20 | 1.35 | 1.50 | / | 1.75 | 2.21 | 2.95 |
| Free-form crystalline form D | 0.00 | 0.40 | 0.85 | 1.34 | 1.85 | 2.30 | 2.76 | / | 3.25 | 3.79 | 4.58 |
| Free-form crystalline form F | 0.00 | 0.18 | 0.38 | 0.60 | 0.84 | 1.14 | 1.54 | / | 2.04 | 2.55 | 3.28 |
| Crystalline form A of disodium salt hydrate | 0.00 | 2.40 | 7.45 | 9.28 | 10.62 | 11.64 | 12.43 | | 13.25 | 14.59 | / |
| Amorphous potassium salt | / | / | / | / | / | / | / | 5.12 | / | 12.98 | 26.64 |

Note: "/" means "no detection".

As can be seen from the above data, the free-form crystalline form C, crystalline form D and crystalline form F were all moderately hygroscopic, and showed no crystalline form changes after DVS testing.

The crystalline form A of disodium salt hydrate and the amorphous potassium salt exhibited comparable hygroscopicity, their weight increased by more than 10% at 80% RH, and they showed no crystalline form changes after DVS testing.

As can be seen from the above experimental results, the free-form crystalline form C has high crystallinity, allows for scale-up preparation, has moderate hygroscopicity, and exhibits extremely high physical and chemical stability under various stability testing conditions. However, the free-form crystalline form C exhibits improved solubility only in a buffer system having a pH of 1.2.

The free-form crystalline form D has moderate crystallinity, allows for scale-up preparation, has moderate hygroscopicity, and exhibits extremely high physical and chemical stability under various stability testing conditions. In addition, the free-form crystalline form D exhibits significantly improved solubility under various pH conditions.

The free-form crystalline form E has moderate crystallinity, and its scale-up preparation is not easy at present.

The free-form crystalline form F has moderate crystallinity, allows for scale-up preparation, has moderate hygroscopicity, and exhibits extremely high physical and chemical stability under various stability testing conditions. In addition, the free-form crystalline form F exhibits extremely significantly improved solubility under various pH conditions.

After salt-forming screening of a large number of basic salts, only crystalline salt forms of the sodium salt, specifically the crystalline form A of disodium salt hydrate and the crystalline form B of anhydrate, were obtained. The sodium salt can be used to prepare salt forms with high crystallinity and allows for scale-up preparation. In addition, under different pH conditions or in various biological media, various crystalline forms of the sodium salt all exhibit significantly higher solubility than the free-form crystalline form C.

Specifically, the crystalline form A of disodium salt hydrate exhibits relatively high physical and chemical stability under various conditions, such as 50 °C, 50 °C/75% RH or brown light exposure, and under high temperature and light exposure, it exhibits relatively high chemical stability but undergoes crystalline form transformation. In further development of formulations or storage and transportation, it is necessary to avoid exposure to light, and the ambient temperature should not be kept too high.

The crystalline form B of disodium salt anhydrate exhibits relatively high physical and chemical stability under various conditions, such as 50 °C, 50 °C/75% RH or brown light exposure; and it exhibits relatively high physical stability under high temperature and high humidity conditions but is sensitive to light. In further development of formulations or storage and transportation, it is necessary to avoid exposure to light.

All documents mentioned in the present invention are incorporated as references, just as each document is individually cited as a reference. In addition, it should be understood that various modifications or changes may be made by those skilled in the art after reading the above teachings of the present invention, and these equivalent forms also fall within the scope to which the present invention relates.

## Claims

1. A crystalline basic salt of a compound of formula (I):

(I)

,

wherein the basic salt is selected from the group consisting of lithium salt, sodium salt, potassium salt, magnesium salt, calcium salt and ammonium salt.

**2.** The crystalline basic salt of the compound of formula (I) according to claim 1, wherein the crystalline basic salt of the compound of formula (I) is a sodium salt; and each molecule of the crystalline sodium salt of the compound of formula (I) comprises the compound of formula (I) in free form and the sodium atom in a molar ratio of 1:1 or 1:2.

**3.** The crystalline basic salt of the compound of formula (I) according to claim 2, wherein each molecule of the crystalline sodium salt of the compound of formula (I) comprises the compound of formula (I) in free form and the sodium atom in a molar ratio of 1:2.

**4.** The crystalline basic salt of the compound of formula (I) according to claim 2, wherein the crystalline sodium salt of the compound of formula (I) is an anhydrate or a hydrate;

**preferably,** each molecule of the crystalline sodium salt hydrate of the compound of formula (I) comprises the compound of formula (I) in free form and the water molecule in a molar ratio of 1.0:(0.1-10.0);
**more preferably,** each molecule of the crystalline sodium salt hydrate of the compound of formula (I) comprises the compound of formula (I) in free form and the water molecule in a molar ratio of 1.0:(1.0-5.0);
**most preferably,** each molecule of the crystalline sodium salt hydrate of the compound of formula (I) comprises the compound of formula (I) in free form and the water molecule in a molar ratio of 1.0:4.0 or 1.0:2.0.

**5.** The crystalline basic salt of the compound of formula (I) according to claim 4, wherein the crystalline basic salt of the compound of formula (I) is a crystalline form A of disodium salt hydrate having an X-ray powder diffraction (XRPD) pattern comprising five or more peaks at angles of diffraction (20) of 8.60±0.2°, 9.97±0.2°, 12.92±0.2°, 15.03±0.2°, 17.62±0.2°, 17.93±0.2°, 20.04±0.2°, 22.07±0.2°, 23.18±0.2°, 23.60±0.2° and 27.06±0.2°;
**preferably,** the X-ray powder diffraction (XRPD) pattern of the crystalline form A of disodium salt hydrate comprises peaks substantially identical to those at angles of diffraction (20) shown in FIG. 1.

**6.** The crystalline basic salt of the compound of formula (I) according to claim 5, wherein a unit cell of the crystalline form A of disodium salt hydrate is a monoclinic crystal system with the following unit cell parameters: a = 20.00(5) Å, b = 5.798(16) Å, c = 20.44(11) Å, $\alpha$ = 90°, $\beta$ = 94.8(2)°, $\gamma$ = 90°, a unit cell volume V of 2362(15) Å3 and space group of C2 (No. 5).

**7.** The crystalline basic salt of the compound of formula (I) according to claim 4, wherein the crystalline basic salt of the compound of formula (I) is a crystalline form B of disodium salt anhydrate having an X-ray powder diffraction (XRPD) pattern comprising five or more peaks at angles of diffraction ($2\theta$) of 6.60±0.2°, 7.46±0.2°, 12.43±0.2°, 13.32±0.2°, 13.63±0.2°, 15.54±0.2°, 17.32±0.2°, 18.68±0.2° and 21.73±0.2°;
**preferably,** the X-ray powder diffraction (XRPD) pattern of the crystalline form B of disodium salt anhydrate comprises peaks substantially identical to those at angles of diffraction ($2\theta$) shown in FIG. 2.

**8.** A preparation method for the crystalline basic salt of the compound of formula (I) according to any one of claims 1-7, comprising the following steps:

1) dissolving or dispersing the compound of formula (I) in free form in water or a suitable organic solvent, and adding a basic solution to the above system to conduct a salt-forming reaction; or adding the compound of formula (I) in free form to a basic solution to conduct a salt-forming reaction; and
2) collecting a solid product precipitated during the above salt-forming reaction, or creating a degree of supersaturation in the salt-forming system to obtain the crystalline basic salt of the compound of formula (I);

wherein the basic salt is selected from the group consisting of lithium salt, sodium salt, potassium salt, magnesium salt, calcium salt and ammonium salt; and
the basic solution is selected from the group consisting of solutions of lithium hydroxide, sodium hydroxide, potassium hydroxide, magnesium hydroxide, calcium hydroxide and ammonia in water or in an organic solvent.

**9.** The preparation method according to claim 8, wherein methods for creating the degree of supersaturation in the salt-forming system in step 2) include one or more of the following: adding a seed crystal, volatilizing the solvent, adding an anti-solvent and cooling.

**10.** The preparation method according to claim 8, wherein the organic solvent is selected from the group consisting of alcohols, chloroalkanes, ketones, ethers, cyclic ethers, esters, alkanes, cycloalkanes, benzenes, amides and sulfoxides, and mixtures thereof and aqueous solutions thereof;
**preferably,** the organic solvent is selected from the group consisting of methanol, ethanol, *n*-propanol, isopropanol, dichloromethane, heptane, acetonitrile, acetone, methyl ethyl ketone, toluene, 1,4-dioxane, tetrahydrofuran, *N*,*N*-dimethylformamide, ethyl acetate, isopropyl acetate, methyl *tert*-butyl ether and 2-methoxyethyl ether, and mixtures thereof and aqueous solutions thereof.

**11.** A preparation method for the crystalline basic salt of the compound of formula (I) according to any one of claims 1-7, comprising the following step: transforming one crystalline form of the basic salt of the compound of formula (I) into another crystalline form of the salt by using a crystalline form transformation method, wherein the crystalline form transformation method includes heating or suspension crystalline form transformation in a solvent selected from the group consisting of methanol, ethanol, *n*-propanol, isopropanol, dichloromethane, heptane, acetonitrile, acetone, methyl ethyl ketone, toluene, 1,4-dioxane, tetrahydrofuran, *N*,*N*-dimethylformamide, ethyl acetate, isopropyl acetate, methyl *tert*-butyl ether and 2-methoxyethyl ether, and mixtures thereof and aqueous solutions thereof.

**12.** A crystalline form of a compound of formula (I) in free form:

(I) ;

**preferably,** the crystalline form of the compound of formula (I) in free form is crystalline form C, crystalline form D, crystalline form E or crystalline form F.

**13.** The crystalline form of the compound of formula (I) in free form according to claim 12, wherein the crystalline form of the compound of formula (I) in free form is the crystalline form C, an X-ray powder diffraction (XRPD) pattern of which comprises five or more peaks at angles of diffraction (2θ) of 7.80±0.2°, 11.97±0.2°, 12.42±0.2°, 15.98±0.2°, 17.37 ±0.2°, 18.82±0.2°, 22.47±0.2°, 23.30±0.2°, 25.01±0.2° and 25.63±0.2°;
**preferably,** the X-ray powder diffraction (XRPD) pattern of the crystalline form C comprises peaks substantially identical to those at angles of diffraction (2θ) shown in FIG. 3.

**14.** The crystalline form of the compound of formula (I) in free form according to claim 12, wherein the crystalline form of the compound of formula (I) in free form is the crystalline form D, an X-ray powder diffraction (XRPD) pattern of which comprises five or more peaks at angles of diffraction (2θ) of 7.97±0.2°, 11.82±0.2°, 12.18±0.2°, 14.87±0.2°, 16.13 ±0.2°, 17.18±0.2°, 19.21±0.2°, 20.72±0.2° and 25.28±0.2°;
**preferably,** the X-ray powder diffraction (XRPD) pattern of the crystalline form D comprises peaks substantially identical to those at angles of diffraction (2θ) shown in FIG. 4.

**15.** The crystalline form of the compound of formula (I) in free form according to claim 12, wherein the crystalline form of the compound of formula (I) in free form is the crystalline form E, an X-ray powder diffraction (XRPD) pattern of which comprises five or more peaks at angles of diffraction (2θ) of 8.16±0.2°, 12.05±0.2°, 12.83±0.2°, 15.19±0.2°, 16.18 ±0.2°, 16.59±0.2°, 17.62±0.2°, 18.41±0.2°, 22.90±0.2° and 25.65±0.2°;

**preferably,** the X-ray powder diffraction (XRPD) pattern of the crystalline form E comprises peaks substantially identical to those at angles of diffraction (2θ) shown in FIG. 5.

**16.** The crystalline form of the compound of formula (I) in free form according to claim 12, wherein the crystalline form of the compound of formula (I) in free form is the crystalline form F, an X-ray powder diffraction (XRPD) pattern of which comprises five or more peaks at angles of diffraction (2θ) of 6.92±0.2°, 9.10±0.2°, 12.42±0.2°, 13.46±0.2°, 14.38 ±0.2°, 15.84±0.2°, 19.04±0.2°, 22.00±0.2° and 26.23±0.2°;
**preferably,** the X-ray powder diffraction (XRPD) pattern of the crystalline form F comprises peaks substantially identical to those at angles of diffraction (2θ) shown in FIG. 6.

**17.** Use of the crystalline form of the compound of formula (I) in free form according to any one of claims 12-16, wherein the crystalline form of the compound of formula (I) in free form is used as a starting material to prepare the crystalline basic salt of the compound of formula (I) according to any one of claims 1-7.

**18.** A pharmaceutical composition, comprising a clinically effective amount of the crystalline basic salt of the compound of formula (I) according to any one of claims 1-7 or the crystalline form of the compound of formula (I) in free form according to any one of claims 12-16 and a pharmaceutically acceptable carrier;
**preferably,** the pharmaceutical composition comprises 0.01-99.0% W/W of the crystalline basic salt of the compound of formula (I) or the crystalline form of the compound of formula (I) in free form relative to a total content of the pharmaceutical composition.

**19.** Use of the crystalline basic salt of the compound of formula (I) according to any one of claims 1-7 or the crystalline form of the compound of formula (I) in free form according to any one of claims 12-16 in preparation of a medicament for treating a PD-1/PD-L1 signal pathway-mediated tumor, immunity-related disease and disorder, communicable disease, infectious disease or metabolic disease;
**preferably,** the tumor is cancer.

**20.** The use according to claim 19, wherein the infectious disease is a bacterial infectious disease, a viral infectious disease or a fungal infectious disease.

**21.** The use according to claim 19, wherein the tumor is lymphoma, sarcoma, melanoma, glioblastoma, synovioma, meningioma, biliary tract tumor, neuroma, seminoma, nephroblastoma, hepatocellular papilloma, papilloma, leiomyoma, rhabdomyoma, hemangioma, lymphangioma, osteoma, lipoma, fibroma, central nervous system tumor, spinal axis tumor, brain stem glioma, multiple myeloma, ovarian tumor, myelodysplastic syndrome or mesothelioma, anal cancer, testicular cancer, urethral carcinoma, penile cancer, bladder cancer, ureteral cancer, uterine cancer, ovarian cancer, fallopian tube cancer, cervical cancer, vaginal cancer, vulvar cancer, Merkel cell carcinoma, embryonal carcinoma, chronic or acute leukemia, bronchial carcinoma, esophageal cancer, nasopharyngeal carcinoma, hepatocellular carcinoma, basal cell carcinoma, lung cancer, adenocarcinoma, papillary carcinoma, rectal cancer, colon cancer, gastric cancer, head and neck cancer, bone cancer, skin cancer, small intestine cancer, endocrine cancer, renal pelvic carcinoma, epidermoid carcinoma, transitional cell carcinoma or choriocarcinoma;

the immunity-related disease and disorder is rheumatic arthritis, renal failure, lupus erythematosus, asthma, psoriasis, ulcerative colitis, pancreatitis, allergy, fibrosis, anemia, fibromyalgia, Alzheimer's disease, congestive heart failure, stroke, aortic valve stenosis, arteriosclerosis, osteoporosis, Parkinson's disease, Crohn's disease, ulcerative colitis, allergic contact dermatitis and eczema, systemic sclerosis or multiple sclerosis;
the communicable disease or infectious disease is sepsis, liver infection, hepatitis A, hepatitis B, hepatitis C, hepatitis D, herpes virus, papillomavirus or influenza; and
the metabolic disease is diabetes, diabetic ketoacidosis, hyperglycemic hyperosmolar syndrome, hypoglycemia, gout, malnutrition, vitamin A deficiency, scurvy, vitamin D deficiency or osteoporosis.

**22.** The use according to claim 21, wherein the lymphoma is lymphocytic lymphoma, primary central nervous system lymphoma, T cell lymphoma, diffuse large B cell lymphoma, follicle center lymphoma, Hodgkin lymphoma, non-Hodgkin lymphoma or primary mediastinal large B cell lymphoma;

the sarcoma is Kaposi's sarcoma, fibrosarcoma, liposarcoma, chondrosarcoma, osteosarcoma, leiomyosarcoma, rhabdomyosarcoma, soft tissue sarcoma, angiosarcoma or lymphangiosarcoma; and
the chronic or acute leukemia is acute myeloid leukemia, chronic myeloid leukemia, acute lymphoblastic leukemia, chronic granulocytic leukemia or chronic lymphoblastic leukemia.

**23.** The use according to claim 19, wherein the tumor is small cell lung cancer, squamous non-small cell lung cancer, non-squamous non-small cell lung cancer, chondroma, colorectal cancer, gastrointestinal cancer, endometrial cancer, head and neck squamous cell carcinoma, abdominal wall carcinoma, renal cell carcinoma or recurrent or existing drug-resistant prostate cancer.

**24.** The use according to claim 19, wherein the tumor is thymic tumor, pleomorphic adenoma, renal tubule adenoma, cystadenoma, pituitary adenoma, prostate cancer, thyroid cancer, parathyroid cancer, adrenal cancer, breast cancer, cystadenocarcinoma or pancreatic cancer.

**25.** The crystalline basic salt of the compound of formula (I) according to any one of claims 1-7 or the crystalline form of the compound of formula (I) in free form according to any one of claims 12-16 for use as a medicament for treating a PD-1/PD-L1 signal pathway-mediated tumor, immunity-related disease and disorder, communicable disease, infectious disease or metabolic disease.

17,000
15,000
14,000
13,000
12,000
11,000
10,000
9,000
8,000
7,000
6,000
5,000
4,000
3,000
2,000
0
10
20
30

FIG. 1

3,000
2,600
2,400
2,200
2,000
1,800
1,600
1,400
1,200
1,000
800
600
400
0
10
20
30
40

FIG. 2

6,000
5,000
4,000
3,000
2,000
1,000
0
10
20
30
40

FIG. 3

4,000
3,000
2,000
1,000
0
10
20
30

FIG. 4

5,000
4,000
3,000
2,000
1,000
0
10
20
30

FIG. 5

6,000
5,000
4,000
3,000
2,000
1,000
0
10
20
30

FIG. 6

c
C
H
N
O
Na

FIG. 7

Weight loss: 0.313 mg
Weight loss percentage: 6.839%
Peak temperature: 258.32 °C
Enthalpy (normalized): 6.9847 J/g
Initial temperature: 237.29 °C
Enthalpy (normalized): 299.60 J/g
Initial temperature: 51.97 °C
Peak temperature: 331.72 °C
Enthalpy (normalized): 14.199 J/g
Initial temperature: 319.33 °C
Peak temperature: 112.38 °C
0.2
0.0
-0.2
-0.4
-0.6
-0.8
-1.0
-1.2
0
100
200
300
400
500
100
80
60
40
20

FIG. 8

Weight loss: 0.075 mg
Weight loss percentage: 2.751%
Enthalpy (normalized): 106.22 J/g
Initial temperature: 41.95 °C
Midpoint type: Half height
Midpoint: 266.84 °C
Enthalpy (normalized): 15.876 J/g
Initial temperature: 316.36 °C
Peak temperature: 92.99 °C
Peak temperature: 330.17 °C
0.6
0.4
0.2
0.0
-0.2
100
80
60
40
20
0
100
200
300
400
500

FIG. 9

Weight loss percentage: 2.0%
Weight loss percentage: 0.7%
Enthalpy (normalized): 60.7 J/g
Initial temperature: 226.7 °C
Enthalpy (normalized): 12.5 J/g
Initial temperature: 169.5 °C
Peak temperature: 177.1 °C
Temperature: 199.2 °C
Peak temperature: 230.5 °C
110
100
90
80
70
2.0
1.2
0.4
-0.4
-1.2
-2.0
0
50
100
150
200
250
300
350

FIG. 10

Weight loss: 0.049 mg
Weight loss percentage: 1.469%
Weight loss: 0.028 mg
Weight loss percentage: 0.858%
Enthalpy (normalized): 41.414 J/g
Initial temperature: Not found
Peak temperature: 66.73 °C
Enthalpy (normalized): 107.07 J/g
Initial temperature: 239.99 °C
Peak temperature: 244.43 °C
1
0
-1
-2
-3
-4
0
50
100
150
200
250
300
100
85.000

FIG. 11

1
0
-1
-2
-3
0
50
100
150
200
250
300
Enthalpy (normalized): 71.925 J/g
Initial temperature: 224.68 °C
Peak temperature: 228.63 °C

FIG. 12

Weight loss: 0.019 mg
Weight loss percentage: 0.653%
Weight loss: 0.019 mg
Weight loss percentage: 0.649%
Enthalpy (normalized): 104.57 J/g
Initial temperature: 221.12 °C
Peak temperature: 67.22 °C
Enthalpy (normalized): 31.985 J/g
Initial temperature: Not found
Peak temperature: 230.50 °C
1
0
-1
-2
-3
$10^2$
0
50
100
150
200
250
300

FIG. 13

Cycle 1 adsorption
Cycle 1 desorption
Cycle 2 adsorption
4.709
2.221
1.941
5.00
4.50
4.00
3.50
3.00
2.50
2.00
1.50
1.00
0.50
0.00
0
10
20
30
40
50
60
70
80
90
100

FIG. 14

Cycle 1 adsorption
Cycle 1 desorption
Cycle 2 adsorption
Cycle 2 desorption
7
6
5
4
3
2
1
0
0
10
20
30
40
50
60
70
80
90
100

FIG. 15

Cycle 1 adsorption
Cycle 1 desorption
Cycle 2 adsorption
Cycle 2 desorption
5
4.5
4
3.5
3
2.5
2
1.5
1
0.5
0
0
10
20
30
40
50
60
70
80
90
100

FIG. 16

Cycle 1 adsorption
Cycle 1 desorption
Cycle 2 adsorption
Cycle 2 desorption
16
14
12
10
8
6
4
2
0
0
10
20
30
40
50
60
70
80
90


FIG. 17

700
600
500
400
300
200
100
0
10
20
30
40

FIG. 18

1000
800
600
400
200
0
10
20
30
40

FIG. 19

**INTERNATIONAL SEARCH REPORT**

International application No.
**PCT/CN2024/129978**

**A. CLASSIFICATION OF SUBJECT MATTER**

C07D401/14(2006.01)i； A61K31/443(2006.01)i； A61K31/444(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： C07D A61K A61P

Documentation searched other than minimum documentation to the extent that such documents are included in the fields searched

Electronic data base consulted during the international search (name of data base and, where practicable, search terms used)

CNABS; CNTXT; DWPI; ENTXTC; WOTXT; USTXT; EPTXT; CNKI; 万方, WANFANG; STN; ISI Web of Science: 上海和誉, 晶体, 联苯基, 哌啶, PD-L1, PD-1, crystal, biphenyl, pyridine, 结构式

**C. DOCUMENTS CONSIDERED TO BE RELEVANT**

| Category* | Citation of document, with indication, where appropriate, of the relevant passages | Relevant to claim No. |
|---|---|---|
| X | WO 2019149183 A1 (ABBISKO THERAPEUTICS CO., LTD.) 08 August 2019 (2019-08-08) description, page 2, line 8-page 20, line 17, and embodiment 110 | 1-25 |
| A | CN 113825751 A (ABBISKO THERAPEUTICS CO., LTD.) 21 December 2021 (2021-12-21) entire document | 1-25 |
| A | CN 115536657 A (SHANGHAI HANSOH BIOMEDICAL CO., LTD.) 30 December 2022 (2022-12-30) entire document | 1-25 |
| A | WO 2023169373 A1 (ABBISKO THERAPEUTICS CO., LTD.) 14 September 2023 (2023-09-14) entire document | 1-25 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 December 2024** | **13 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/129978**

| Patent document cited in search report | Publication date (day/month/year) | Patent family member(s) | Publication date (day/month/year) |
|---|---|---|---|
| WO 2019149183 A1 | 08 August 2019 | CA 3088927 A1 | 08 August 2019 |
| | | CA 3088927 C | 21 March 2023 |
| | | KR 20200100717 A | 26 August 2020 |
| | | KR 102534185 B1 | 18 May 2023 |
| | | TW 201934544 A | 01 September 2019 |
| | | EP 3750887 A1 | 16 December 2020 |
| | | EP 3750887 A4 | 20 October 2021 |
| | | BR 112020014459 A2 | 15 December 2020 |
| | | AU 2019214089 A1 | 09 July 2020 |
| | | AU 2019214089 B2 | 11 February 2021 |
| | | JP 2021512878 A | 20 May 2021 |
| | | JP 7033343 B2 | 10 March 2022 |
| | | PH 12020551184 A1 | 10 May 2021 |
| | | US 2021032270 A1 | 04 February 2021 |
| | | US 11459339 B2 | 04 October 2022 |
| | | CN 111448189 A | 24 July 2020 |
| | | CN 111448189 B | 10 September 2024 |
| | | HK 40023971 A0 | 04 December 2020 |
| | | TW 828649 B1 | 11 January 2024 |
| | | MX 398873 B | 04 January 2023 |
| | | SG 11202006409 A1 | 28 August 2020 |
| | | RU 2020127950 A | 11 March 2022 |
| | | RU 2768830 C2 | 24 March 2022 |
| | | RU 2768830 C9 | 17 June 2022 |
| CN 113825751 A | 21 December 2021 | US 2022380342 A1 | 01 December 2022 |
| | | WO 2021008491 A1 | 21 January 2021 |
| | | EP 4001274 A1 | 25 May 2022 |
| | | EP 4001274 A4 | 26 July 2023 |
| CN 115536657 A | 30 December 2022 | None | |
| WO 2023169373 A1 | 14 September 2023 | TW 202340165 A | 16 October 2023 |
| | | AU 2023232372 A1 | 04 July 2024 |
| | | KR 20240110977 A | 16 July 2024 |
| | | CA 3242373 A1 | 14 September 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019149183 A1